# EUROPEAN PATENT APPLICATION

(11) **EP 4 681 736 A2**
(43) Date of publication of application: **21.01.2026**
(21) Application number: 25220163.7
(22) Date of filing: 05.08.2021
(51) Int. Cl.: A61K 47/02

(54) **METHODS OF TREATMENT USING FUROSEMIDE**

(30) Priority: 05.08.2020 US 202063061518 P
(62) Divisional of application: 21770335.4
(71) Applicant: scPharmaceuticals Inc., Burlington, MA 01803 (US)
(72) Inventor: MOHR, John, F., Acton, 01720 (US); TUCKER, John, Rye, 03871 (US); HASSMAN, Michael, Foxborough, 02035 (US); PECORELLI, Erik, Mark, Hartford, 06107 (US)
(74) Representative: Graham Watt & Co

(57) **Abstract**

Disclosed herein, in part, are methods of subcutaneously administering liquid pharmaceutical formulations of furosemide to an adult patient from a prefilled cartridge using a five-hour bi-phasic delivery profile. Methods of treating congestion, edema, fluid overload, or hypertension in a patient in need thereof are also provided.

## Description

### CROSS-REFERENCE TO RELATED APPLICATION

This application claims the benefit of, and priority to, U.S. Provisional Application No. 63/061,518, filed on August 5, 2020, the content of which is hereby incorporated by reference in its entirety.

### BACKGROUND

Furosemide, an exemplary loop diuretic, can be used in the treatment of hypertension, edema and related conditions, including decompensated heart failure. Furosemide is commonly used in the treatment and/or management of edema associated with cardiac, renal, and hepatic insufficiency or failure, for example, congestive heart failure. H. Bundgaard, T. Norgaard, N. M. Nielsen, "Photodegradation and hydrolysis of furosemide and furosemide esters in aqueous solutions, " International Journal of Pharmaceutics 42, 217 (1988).

Oral bioavailability, and therefore oral efficacy, of furosemide is limited. Furosemide is commonly administered both parenterally and orally, although highly variable oral absorption is observed due to the combined effects of limited solubility and decreased stability at acidic pH. B. Devarakonda, D. P. Otto, A. Judefeind, R. A. Hill, M. M. de Villiers, "Effect of pH on the solubility and release of furosemide from polyamidoamine (PAMAM) dendrimer complexes, " International Journal of Pharmaceutics 345, 142 (Dec. 10, 2007). Accordingly, furosemide typically is administered intravenously or intramuscularly for most patients with decompensated heart failure or other forms of more advanced edema.

Intravenous administration of a pharmaceutical drug, such as furosemide, requires a trained healthcare professional for placement of the catheter and administration of the drug solution. In contrast, subcutaneous administration of a pharmaceutical drug can be accomplished with the aid of auto-injection devices and/or minipumps or subcutaneous injections or infusions, which can permit administration to be performed by the patient or caregiver, for example, at home.

For subcutaneous administration, discomfort and pain during administration should be minimized so as to avoid poor patient compliance with the treatment regimen. Factors that can contribute to pain and discomfort perceived by a patient upon, during, or after subcutaneous administration include the injection volume, the pH of the formulation, and the osmoticity or tonicity of the formulation. Moreover, such a formulation should be stable in solution so that it readily is available for use and/or can be pre-loaded into a variety of dispensing devices.

Thus, the art desires new and alternative formulations and delivery vehicles and profiles for the subcutaneous administration of furosemide at therapeutically effective amounts.

### SUMMARY

In one aspect, the invention provides methods of treating congestion due to fluid overload in an adult human in need thereof, wherein the method generally comprises administering a liquid pharmaceutical formulation comprising furosemide. In some embodiments, a therapeutically effective amount of furosemide is subcutaneously administered, for example, over a five-hour time period, to produce one or more of the results or outcomes described herein, for example, the furosemide concentration in plasma of the adult human is equal to or greater than 1000 ng/mL after the first hour of administration to about one hour after administration is complete. In some embodiments, the methods include administering a liquid pharmaceutical formulation including furosemide, wherein the liquid pharmaceutical formulation is contained in a prefilled cartridge, for example, a prefilled polymeric cartridge.

In various embodiments of the invention, the method comprises administering subcutaneously a liquid pharmaceutical formulation from a prefilled polymeric cartridge to the adult human over about 5 hours, wherein
the liquid pharmaceutical formulation comprises about 80 mg furosemide;
a total volume of about 10 mL of the liquid pharmaceutical formulation is administered over about 5 hours; and
the furosemide concentration in plasma of the adult human is about 1200 ng/mL to about 1850 ng/mL after the first hour of administration, and about 1000 ng/mL to about 1800 ng/mL about one hour after administration is complete.

In various embodiments of the invention, the method comprises administering subcutaneously a liquid pharmaceutical formulation from a prefilled polymeric cartridge to the adult human over about 5 hours, wherein
the liquid pharmaceutical formulation comprises about 80 mg furosemide;
a total volume of about 10 mL of the liquid pharmaceutical formulation is administered over about 5 hours; and
one or more of the following:
   the furosemide concentration in plasma of the adult human is about 1200 ng/mL to about 1850 ng/mL after the first hour of administration, and about 1000 ng/mL to about 1800 ng/mL about one hour after administration is complete;
   the total urine output of the adult human during the first hour of administration is about 150 mL to about 450 mL and the average hourly urine output of the adult human one hour after administration is complete is about 250 mL to about 550 mL;
   the total urine output of the adult human about one hour after administration is complete is about 1500 mL to about 4000 mL;
   the total urine sodium excretion about one hour after administration is complete is about 375 mmol to about 500 mmol;
   the maximum total urine output of the adult human about one hour after administration is complete is about 2800 mL to about 4000 mL; and
   the maximum total urine sodium excretion about one hour after administration is complete is about 450 mmol to about 550 mmol.

In various embodiments of the invention, the method comprises administering subcutaneously a liquid pharmaceutical formulation from a prefilled polymeric cartridge to the adult human over about 5 hours, wherein
the liquid pharmaceutical formulation comprises about 80 mg furosemide;
a total volume of about 10 mL of the liquid pharmaceutical formulation is administered over about 5 hours;
the furosemide concentration in plasma of the adult human is about 1200 ng/mL to about 1850 ng/mL after the first hour of administration, and about 1000 ng/mL to about 1800 ng/mL about one hour after administration is complete;
the total urine output of the adult human during the first hour of administration is about 150 mL to about 450 mL and the average hourly urine output of the adult human one hour after administration is complete is about 250 mL to about 550 mL;
the total urine output of the adult human about one hour after administration is complete is about 1500 mL to about 4000 mL;
the total urine sodium excretion about one hour after administration is complete is about 375 mmol to about 500 mmol;
the maximum total urine output of the adult human about one hour after administration is complete is about 2800 mL to about 4000 mL; and
the maximum total urine sodium excretion about one hour after administration is complete is about 450 mmol to about 550 mmol.

In various embodiments of the invention, the method comprises administering subcutaneously a liquid pharmaceutical formulation from a prefilled polymeric cartridge to the adult human using a five-hour bi-phasic delivery profile, wherein
the liquid pharmaceutical formulation comprises about 80 mg furosemide, about 79 mg tromethamine hydrochloride, and water, and has a pH between about 7 to about 7.8 and a total volume of 10 mL;
the five-hour bi-phasic delivery profile administers about 30 mg furosemide over the first hour and about 12.5 mg furosemide over each of the subsequent four hours, wherein the total volume of 10 mL of the liquid pharmaceutical formulation is administered over about five hours: and
one or more of the following:
   the furosemide concentration in plasma of the adult human is about 1200 ng/mL to about 1850 ng/mL after the first hour of administration, and about 1000 ng/mL to about 1800 ng/mL about one hour after administration is complete;
   the total urine output of the adult human during the first hour of administration is about 150 mL to about 450 mL and the average hourly urine output of the adult human one hour after administration is complete is about 250 mL to about 550 mL;
   the total urine output of the adult human about one hour after administration is complete is about 1500 mL to about 4000 mL;
   the total urine sodium excretion about one hour after administration is complete is about 375 mmol to about 500 mmol;
   the maximum total urine output of the adult human about one hour after administration is complete is about 2800 mL to about 4000 mL; and
   the maximum total urine sodium excretion about one hour after administration is complete is about 450 mmol to about 550 mmol.

In various embodiments of the invention, the method comprises administering subcutaneously a liquid pharmaceutical formulation from a prefilled polymeric cartridge to the adult human using a five-hour bi-phasic delivery profile, wherein
the liquid pharmaceutical formulation comprises about 80 mg furosemide, about 79 mg tromethamine hydrochloride, and water, and has a pH between about 7 to about 7.8 and a total volume of 10 mL;
the five-hour bi-phasic delivery profile administers about 30 mg furosemide over the first hour and about 12.5 mg furosemide over each of the subsequent four hours, wherein the total volume of 10 mL of the liquid pharmaceutical formulation is administered over about five hours;
the furosemide concentration in plasma of the adult human is about 1200 ng/mL to about 1850 ng/mL after the first hour of administration, and about 1000 ng/mL to about 1800 ng/mL about one hour after administration is complete;
the total urine output of the adult human during the first hour of administration is about 150 mL to about 450 mL and the average hourly urine output of the adult human one hour after administration is complete is about 250 mL to about 550 mL;
the total urine output of the adult human about one hour after administration is complete is about 1500 mL to about 4000 mL;
the total urine sodium excretion about one hour after administration is complete is about 375 mmol to about 500 mmol;
the maximum total urine output of the adult human about one hour after administration is complete is about 2800 mL to about 4000 mL; and
the maximum total urine sodium excretion about one hour after administration is complete is about 450 mmol to about 550 mmol.

In another aspect, the invention provides a single-use polymeric prefilled cartridge that can be associated with an on-body wearable delivery device using a five-hour bi-phasic delivery profile for administering subcutaneously a liquid pharmaceutical formulation to an adult human.

In various embodiments, the single-use polymeric prefilled cartridge comprises the liquid pharmaceutical formulation and the liquid pharmaceutical formulation is isosmotic, has a pH between about 7 to about 7.8, and consists of:
about 80 mg furosemide;
about 79 mg tromethamine hydrochloride:
optionally, one or more other pharmaceutically acceptable excipients; and
water to a total volume of 10 mL.

### BRIEF DESCRIPTION OF THE DRAWINGS

FIG. 1 is a plot depicting the effect of furosemide concentration (ng/mL) in plasma on average hourly urine output (mL), as further described in Example 2.
FIG. 2 is a plot depicting the effect of furosemide concentration (ng/mL) in plasma on average hourly urine sodium excretion (mL), as further described in Example 2.

### DETAILED DESCRIPTION

As generally described herein, the invention provides methods of treating various conditions, diseases, and disorders (e.g., congestion due to fluid overload) in an adult patient (human) in need thereof. The methods generally can comprise subcutaneously administering a liquid pharmaceutical formulation of furosemide to the adult patient from a prefilled polymeric cartridge using a five-hour delivery profile. The liquid pharmaceutical formulations of furosemide and delivery profiles described herein can result in optimized furosemide pharmacokinetic and pharmacodynamic profiles that maximize patient outcomes (e.g., total urine output and urine sodium excretion).

### Definitions

To facilitate an understanding of the present invention, a number of terms and phrases are defined below.

Unless defined otherwise, all technical and scientific terms used herein have the same meaning as commonly understood by one of ordinary skill in the art to which this invention belongs. The abbreviations used herein have their conventional meaning within the chemical and biological arts. The chemical structures and formulae set forth herein are constructed according to the standard rules of chemical valency known in the chemical arts.

The terms "a" and "an" as used herein mean "one or more" and include the plural unless the context is inappropriate.

In the application, where an element or component is said to be included in and/or selected from a list of recited elements or components, it should be understood that the element or component can be any one of the recited elements or components, or the element or component can be selected from a group consisting of two or more of the recited elements or components.

Further, it should be understood that elements and/or features of a composition or a method described herein can be combined in a variety of ways without departing from the spirit and scope of the present invention, whether explicit or implicit herein. For example, where reference is made to a particular compound, that compound can be used in various embodiments of compositions of the present invention and/or in methods of the present invention, unless otherwise understood from the context. In other words, within this application, embodiments have been described and depicted in a way that enables a clear and concise application to be written and drawn, but it is intended and will be appreciated that embodiments may be variously combined or separated without parting from the present teachings and invention(s). For example, it will be appreciated that all features described and depicted herein can be applicable to all aspects of the invention(s) described and depicted herein.

It should be understood that the expression "at least one of" includes individually each of the recited objects after the expression and the various combinations of two or more of the recited objects unless otherwise understood from the context and use. The expression "and/or" in connection with three or more recited objects should be understood to have the same meaning unless otherwise understood from the context.

The use of the term "include," "includes," "including," "have," "has," "having," "contain," "contains," or "containing," including grammatical equivalents thereof, should be understood generally as open-ended and non-limiting, for example, not excluding additional unrecited elements or steps, unless otherwise specifically stated or understood from the context.

Where the use of the term "about" is before a quantitative value, the present invention also includes the specific quantitative value itself, unless specifically stated otherwise. As used herein, the term "about" refers to a ±10% variation from the nominal value unless otherwise indicated or inferred from the context.

At various places in the present specification, values are disclosed in groups or in ranges. It is specifically intended that the description include each and every individual subcombination of the members of such groups and ranges. For example, an integer in the range of 0 to 40 is specifically intended to individually disclose 0, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, and 40, and an integer in the range of 1 to 20 is specifically intended to individually disclose 1, 2, 3, 4, 5, 6, 7, 8, 9, 10, 11, 12, 13, 14, 15, 16, 17, 18, 19, and 20.

The use of any and all examples, or exemplary language herein, for example, "such as" or "including," is intended merely to illustrate better the present invention and does not pose a limitation on the scope of the invention unless claimed. No language in the specification should be construed as indicating any non-claimed element as essential to the practice of the present invention.

As used herein, a "compound" (including a specifically named compound, e.g., furosemide) refers to the compound itself and its pharmaceutically acceptable salts unless otherwise understood from the context of the description or expressly limited to one particular form of the compound, e.g., the compound itself, or a pharmaceutically acceptable salt thereof.

As used herein, "furosemide" refers to a compound having the formula: 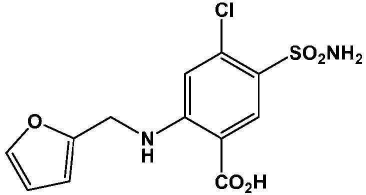 and pharmaceutically acceptable salts thereof. Such salts may include, but are not limited to, furosemide sodium salt and furosemide quaternary ammonium salt. Furosemide may be referred to by other names, for example, frusemide, 5-(aminosulphonyl)-4-chloro-2-[(2-furanyl-methyl)amino]benzoic acid, or its IUPAC name, 4-chloro-2-(furan-2-ylmethylamino)-5-sulfamoyl-benzoic acid, or its common trade name, Lasix^{®}.

As used herein, the terms "subject" and "patient" refer to organisms to be treated by the methods and/or compositions described herein. Such organisms are preferably mammals (*e*.*g*., murines, simians, equines, bovines, porcines, canines, felines, and the like), and more preferably humans.

As used herein, a "buffer" refers to an aqueous solution that is resistant to changes in pH. A buffer may include a "buffering agent" such as a weak acid and its salt, or a weak base and its salt, which assist in maintaining the stability of the pH. Examples of buffers used in pharmaceutical formulations include bicarbonate buffers, carbonate buffers, citrate buffers, histidine buffers, phosphate buffers, tartrate buffers, tris(hydroxymethyl)aminomethane (or 2-amino-2-hydroxymethyl-propane-1,3-diol [(HOCH₂)₃CNH₂]) buffers, and combinations thereof. Certain of these buffers are suitable for pharmaceutical formulations administered subcutaneously.

Tris(hydroxymethyl)aminomethane or a tris(hydroxymethyl)aminomethane buffer can be referred to as "TRIS," "Tris," "Tris buffer," "Trisamine," "THAM," "tromethamine," and other names. In addition, many buffers and/or buffer systems can include Tris, or a pharmaceutically acceptable salt thereof, and can be used in the present teachings. For example, Tris-buffered saline ("TBS"), Tris-hydrochloride buffer ("Tris-HCl"), Tris base (pH 10.6), Tris/borate/ethylene diamine tetra-acetate ("EDTA") buffer ("TBE"), and Tris/acetate/EDTA buffer ("TAE"). Tris base often is used with Tris-HCl to prepare Tris buffers at a desired pH. In addition, the present teachings can include Tris-related compounds, for example, compounds derived from Tris or structurally-related to Tris, that can act as a buffer.

As used herein, "tonicity" refers to the ionic strength or concentration of ions in a solution such as a pharmaceutical formulation. Tonicity often is measured in molarity ("M"). As used herein, an "isotonic solution," an "isotonic formulation," an "isotonic pharmaceutical formulation," and a pharmaceutical formulation that is "isotonic" refers to a solution or formulation that has the same or similar concentration of ions as found in bodily fluids.

As used herein, "physiological pH" refers to a pH of about 7.4.

As used herein, "osmoticity" and "osmolality" refer to the osmotic pressure of a solution such as a pharmaceutical formulation. Osmoticity often is measured in osmolarity ("Osm/L" or "OsM") or osmolality ("Osm/kg"), which can be used interchangeably herein. When measuring freezing point depression, the observed value is the osmolality of the solution. In contrast to tonicity, osmoticity accounts for un-ionized solutes in a solution such that when present, the osmolarity or osmolality of the solution will be higher than its tonicity. The osmolarity of a liquid pharmaceutical formulation described herein can be measured, for example, using a vapor pressure method.

As used herein, an "isosmotic solution," an "isosmotic formulation," an "isosmotic pharmaceutical formulation," and a pharmaceutical formulation that is "isosmotic" refers to a solution or a formulation that has the same or similar concentration of solutes as found in bodily fluids. In certain embodiments, a liquid pharmaceutical formulation that is "isosmotic" can have an osmolarity in the range of about 275 mOsM to about 350 mOsM or when the osmolality of the formulation is in the range of about 275 mOsm/kg to about 350 mOsm/kg.

As used herein, "osmolarity adjustor" and "osmotic agent" refer to a pharmaceutically acceptable compound that may be added to a liquid pharmaceutical formulation described herein in order to modulate the osmolarity of the liquid pharmaceutical formulation.

As used herein, "pharmaceutically acceptable" refers to a substance that is acceptable for use in pharmaceutical applications from a toxicological perspective and does not adversely interact with the active ingredient. Accordingly, pharmaceutically acceptable carriers are those that are compatible with the other ingredients in the formulation and are biologically acceptable. In certain embodiments, supplementary active ingredients can also be incorporated into the pharmaceutical compositions.

As used herein, "pharmaceutically acceptable excipient" refers to a substance that aids the administration of an active agent to and absorption by a subject and can be included in the compositions of the present invention without causing a significant adverse toxicological effect on the patient. Non-limiting examples of pharmaceutically acceptable excipients include water, NaCl, normal saline solutions, a phosphate buffered saline solution, emulsions (e.g., such as an oil/water or water/oil emulsions), lactated Ringer's, normal sucrose, normal glucose, binders, fillers, disintegrants, lubricants, coatings, sweeteners, flavors, salt solutions (such as Ringer's solution), alcohols, oils, gelatins, carbohydrates such as lactose, amylose or starch, fatty acid esters, hydroxypropylmethylcellulose, polyvinyl pyrrolidine, and colors, and the like. Such preparations can be sterilized and, if desired, mixed with auxiliary agents such as lubricants, preservatives, stabilizers, wetting agents, emulsifiers, salts for influencing osmotic pressure, buffers, coloring, and/or aromatic substances and the like that do not deleteriously react with the compounds of the invention. For examples of excipients and carriers, *see* Martin, Remington's Pharmaceutical Sciences, 15th Ed., Mack Publ. Co., Easton, PA (1975).

As used herein, the term "pharmaceutically acceptable carrier" refers to any of the standard pharmaceutical carriers, such as a phosphate buffered saline solution, water, emulsions (*e.g.,* such as an oil/water or water/oil emulsions), and various types of wetting agents. The compositions also can include stabilizers and preservatives. For examples of carriers, stabilizers and adjuvants, see Martin, Remington's Pharmaceutical Sciences, 15th Ed., Mack Publ. Co., Easton, PA [1975].

As used herein, the term "pharmaceutically acceptable salt" refers to any pharmaceutically acceptable salt (*e.g.*, acid or base) of a compound of the present invention which, upon administration to a subject, is capable of providing a compound of this invention or an active metabolite or residue thereof. As is known to those of skill in the art, "salts" of the compounds of the present invention may be derived from inorganic or organic acids and bases. Examples of acids include, but are not limited to, hydrochloric, hydrobromic, sulfuric, nitric, perchloric, fumaric, maleic, phosphoric, glycolic, lactic, salicylic, succinic, toluene-p-sulfonic, tartaric, acetic, citric, methanesulfonic, ethanesulfonic, formic, benzoic, malonic, naphthalene-2-sulfonic, benzenesulfonic acid, and the like. Other acids, such as oxalic, while not in themselves pharmaceutically acceptable, may be employed in the preparation of salts useful as intermediates in obtaining the compounds of the invention and their pharmaceutically acceptable acid addition salts.

Examples of bases include, but are not limited to, alkali metal (*e.g*., sodium) hydroxides, alkaline earth metal (*e.g.*, magnesium) hydroxides, ammonia, and compounds of formula NW₄⁺, wherein W is C₁₋₄ alkyl, and the like.

Examples of salts include, but are not limited to: acetate, adipate, alginate, aspartate, benzoate, benzenesulfonate, bisulfate, butyrate, citrate, camphorate, camphorsulfonate, cyclopentanepropionate, digluconate, dodecylsulfate, ethanesulfonate, fumarate, glucoheptanoate, glycerophosphate, hemisulfate, heptanoate, hexanoate, hydrochloride, hydrobromide, hydroiodide, 2-hydroxyethanesulfonate, lactate, maleate, methanesulfonate, 2-naphthalenesulfonate, nicotinate, oxalate, palmoate, pectinate, persulfate, phenylpropionate, picrate, pivalate, propionate, succinate, tartrate, thiocyanate, tosylate, undecanoate, and the like. Other examples of salts include anions of the compounds of the present invention compounded with a suitable cation such as Na⁺, NH₄⁺, and NW₄⁺ (wherein W is a C₁₋₄ alkyl group), and the like.

As used herein, the term "effective amount" refers to the amount of a composition (*e.g.,* a liquid pharmaceutical formulation of the present invention) sufficient to effect beneficial or desired results. An effective amount can be administered in one or more administrations, applications or dosages and is not intended to be limited to a particular formulation or administration route.

As used herein, the terms "treat," "treating," and "treatment" include any effect, *e.g.,* lessening, reducing, modulating, ameliorating or eliminating, that results in the improvement of the condition, disease, disorder, and the like, or ameliorating a symptom thereof.

The phrase "therapeutically-effective amount" as used herein means that amount of a composition (*e.g*., a liquid pharmaceutical formulation of the present invention) which is effective for producing some desired therapeutic effect in a subject.

As used herein, the term "congestion" (in heart failure) is the presence of signs and symptoms of extracellular fluid accumulation that results in increased cardiac filling pressures leading to reduced cardiac output. This reduced cardiac output is further exacerbated by neurohormonal activation leading to increased renal sodium and water avidity resulting in an increased plasma volume.

As used herein, "fluid overload," "volume overload" and "hypervolemia", may describe a medical condition where there is too much fluid in the blood. Excess fluid, primarily salt and water, may build up throughout the body resulting in weight gain.

Throughout the description, where compositions and kits are described as having, including, or comprising specific components, or where processes and methods are described as having, including, or comprising specific steps, it is contemplated that, additionally, there are compositions and kits of the present invention that consist essentially of, or consist of, the recited components, and that there are processes and methods according to the present invention that consist essentially of, or consist of, the recited processing steps.

As a general matter, compositions specifying a percentage are by weight unless otherwise specified. Further, if a variable is not accompanied by a definition, then the previous definition of the variable controls.

### Liquid Pharmaceutical Formulations of Furosemide

As described herein, in one aspect, the invention provides liquid pharmaceutical formulations comprising furosemide, or a pharmaceutically acceptable salt thereof, for the treatment of a condition, disease, or disorder described herein. In various embodiments, the condition, disease, or disorder is selected from the group consisting of congestion, edema, fluid overload, hypertension, and combinations thereof. In certain embodiments, the condition, disease, or disorder is congestion due to fluid overload.

In various embodiments, the liquid pharmaceutical formulations described herein can comprise about 60 mg to about 100 mg, about 65 mg to about 100 mg, about 70 mg to about 100 mg, about 75 mg to about 100 mg, about 80 mg to about 100 mg, about 85 mg to about 100 mg, about 90 mg to about 100 mg, about 95 mg to about 100 mg, about 60 mg to about 95 mg, about 60 mg to about 90 mg, about 60 mg to about 85 mg, about 60 mg to about 80 mg, about 60 mg to about 75 mg, about 60 mg to about 70 mg, about 60 mg to about 65 mg, about 65 mg to about 95 mg, about 65 mg to about 90 mg, about 65 mg to about 85 mg, about 65 mg to about 80 mg, about 65 mg to about 75 mg, about 65 mg to about 70 mg, about 70 mg to about 95 mg, about 70 mg to about 90 mg, about 70 mg to about 85 mg, about 70 mg to about 80 mg, about 70 mg to about 75 mg, about 75 mg to about 95 mg, about 75 mg to about 90 mg, about 75 mg to about 85 mg, about 75 mg to about 80 mg, about 80 mg to about 95 mg, about 80 mg to about 90 mg, about 80 mg to about 85 mg, about 85 mg to about 95 mg, about 85 mg to about 90 mg, or about 90 mg to about 95 mg furosemide.

In various embodiments, the liquid pharmaceutical formulations described herein can comprise about 60 mg, about 65 mg, about 70 mg, about 75 mg, about 80 mg, about 85 mg, about 90 mg, about 95 mg, or about 100 mg furosemide. In certain embodiments, the liquid pharmaceutical formulations comprise about 80 mg furosemide.

In various embodiments, the liquid pharmaceutical formulations described herein can comprise a pharmaceutically acceptable salt of furosemide.

In various embodiments, a pharmaceutically acceptable salt of furosemide described herein can be present in the liquid pharmaceutical formulations in an amount that provides about 60 mg to about 100 mg, about 65 mg to about 100 mg, about 70 mg to about 100 mg, about 75 mg to about 100 mg, about 80 mg to about 100 mg, about 85 mg to about 100 mg, about 90 mg to about 100 mg, about 95 mg to about 100 mg, about 60 mg to about 95 mg, about 60 mg to about 90 mg, about 60 mg to about 85 mg, about 60 mg to about 80 mg, about 60 mg to about 75 mg, about 60 mg to about 70 mg, about 60 mg to about 65 mg, about 65 mg to about 95 mg, about 65 mg to about 90 mg, about 65 mg to about 85 mg, about 65 mg to about 80 mg, about 65 mg to about 75 mg, about 65 mg to about 70 mg, about 70 mg to about 95 mg, about 70 mg to about 90 mg, about 70 mg to about 85 mg, about 70 mg to about 80 mg, about 70 mg to about 75 mg, about 75 mg to about 95 mg, about 75 mg to about 90 mg, about 75 mg to about 85 mg, about 75 mg to about 80 mg, about 80 mg to about 95 mg, about 80 mg to about 90 mg, about 80 mg to about 85 mg, about 85 mg to about 95 mg, about 85 mg to about 90 mg, or about 90 mg to about 95 mg furosemide in its free base form.

In various embodiments, a pharmaceutically acceptable salt of furosemide described herein can be present in the liquid pharmaceutical formulations in an amount that provides about 60 mg, about 65 mg, about 70 mg, about 75 mg, about 80 mg, about 85 mg, about 90 mg, about 95 mg, or about 100 mg furosemide in its free base form. In certain embodiments, the pharmaceutically acceptable salt of furosemide is present in the liquid pharmaceuticals formulation in an amount that provides about 80 mg furosemide in its free base form.

In various embodiments, the concentration of furosemide in the liquid pharmaceutical formulations described herein can be about 5 mg/mL to about 15 mg/mL, about 6 mg/mL to about 15 mg/mL, about 7 mg/mL to about 15 mg/mL, about 8 mg/mL to about 15 mg/mL, about 9 mg/mL to about 15 mg/mL, about 10 mg/mL to about 15 mg/mL, about 11 mg/mL to about 15 mg/mL, about 12 mg/mL to about 15 mg/mL, about 13 mg/mL to about 15 mg/mL, about 14 mg/mL to about 15 mg/mL, about 5 mg/mL to about 14 mg/mL, about 5 mg/mL to about 13 mg/mL, about 5 mg/mL to about 12 mg/mL, about 5 mg/mL to about 11 mg/mL, about 5 mg/mL to about 10 mg/mL, about 5 mg/mL to about 9 mg/mL, about 5 mg/mL to about 8 mg/mL, about 5 mg/mL to about 7 mg/mL, about 5 mg/mL to about 6 mg/mL, about 6 mg/mL to about 14 mg/mL, about 6 mg/mL to about 13 mg/mL, about 6 mg/mL to about 12 mg/mL, about 6 mg/mL to about 11 mg/mL, about 6 mg/mL to about 10 mg/mL, about 6 mg/mL to about 9 mg/mL, about 6 mg/mL to about 8 mg/mL, about 6 mg/mL to about 7 mg/mL, about 7 mg/mL to about 14 mg/mL, about 7 mg/mL to about 13 mg/mL, about 7 mg/mL to about 12 mg/mL, about 7 mg/mL to about 11 mg/mL, about 7 mg/mL to about 10 mg/mL, about 7 mg/mL to about 9 mg/mL, about 7 mg/mL to about 8 mg/mL, about 8 mg/mL to about 14 mg/mL, about 8 mg/mL to about 13 mg/mL, about 8 mg/mL to about 12 mg/mL, about 8 mg/mL to about 11 mg/mL, about 8 mg/mL to about 10 mg/mL, about 8 mg/mL to about 9 mg/mL, about 9 mg/mL to about 14 mg/mL, about 9 mg/mL to about 13 mg/mL, about 9 mg/mL to about 12 mg/mL, about 9 mg/mL to about 11 mg/mL, about 9 mg/mL to about 10 mg/mL, about 10 mg/mL to about 14 mg/mL, about 10 mg/mL to about 13 mg/mL, about 10 mg/mL to about 12 mg/mL, about 10 mg/mL to about 11 mg/mL, about 11 mg/mL to about 14 mg/mL, about 11 mg/mL to about 13 mg/mL, about 11 mg/mL to about 12 mg/mL, about 12 mg/mL to about 14 mg/mL, about 12 mg/mL to about 13 mg/mL, or about 13 mg/mL to about 14 mg/mL.

In various embodiments, the concentration of furosemide in the liquid pharmaceutical formulations described herein can be about 5 mg/mL, about 6 mg/mL, about 7 mg/mL, about 8 mg/mL, about 9 mg/mL, about 10 mg/mL, about 11 mg/mL, about 12 mg/mL, about 13 mg/mL, about 14 mg/mL, or about 15 mg/mL. In certain embodiments, the concentration of furosemide in the liquid pharmaceutical formulation is about 8 mg/mL.

In various embodiments, the liquid pharmaceutical formulations described herein may further comprise a pharmaceutically acceptable buffer.

In various embodiments, a liquid pharmaceutical composition comprises:
(i) furosemide, or a pharmaceutically acceptable salt thereof; and
(ii) a pharmaceutically acceptable buffer.

In certain embodiments, the pharmaceutically acceptable buffer comprises a buffering agent selected from the group consisting of histidine, a citrate salt, sodium phosphate, potassium phosphate, tromethamine or a pharmaceutically acceptable salt thereof, and combinations thereof. In certain embodiments, the buffering agent is tromethamine, or a pharmaceutically acceptable salt thereof. In certain embodiments, the pharmaceutically acceptable salt of tromethamine is tromethamine hydrochloride.

In various embodiments, the liquid pharmaceutical formulations described herein may further comprise water.

In various embodiments, a liquid pharmaceutical formulation comprises:
(i) furosemide, or a pharmaceutically acceptable salt thereof;
(ii) a pharmaceutically acceptable buffer: and
(iii) water.

In various embodiments, the liquid pharmaceutical formulations described herein can comprise about 75 mg to about 85 mg, about 76 mg to about 85 mg, about 77 mg to about 85 mg, about 78 mg to about 85 mg, about 79 mg to about 85 mg, about 80 mg to about 85 mg, about 81 mg to about 85 mg, about 82 mg to about 85 mg, about 83 mg to about 85 mg, about 84 mg to about 85 mg, about 75 mg to about 84 mg, about 75 mg to about 83 mg, about 75 mg to about 82 mg, about 75 mg to about 81 mg, about 75 mg to about 80 mg, about 75 mg to about 79 mg, about 75 mg to about 78 mg, about 75 mg to about 77 mg, about 75 mg to about 76 mg, about 76 mg to about 84 mg, about 76 mg to about 83 mg, about 76 mg to about 82 mg, about 76 mg to about 81 mg, about 76 mg to about 80 mg, about 76 mg to about 79 mg, about 76 mg to about 78 mg, about 76 mg to about 77 mg, about 77 mg to about 84 mg, about 77 mg to about 83 mg, about 77 mg to about 82 mg, about 77 mg to about 81 mg, about 77 mg to about 80 mg, about 77 mg to about 79 mg, about 77 mg to about 78 mg, about 78 mg to about 84 mg, about 78 mg to about 83 mg, about 78 mg to about 82 mg, about 78 mg to about 81 mg, about 78 mg to about 80 mg, about 78 mg to about 79 mg, about 79 mg to about 84 mg, about 79 mg to about 83 mg, about 79 mg to about 82 mg, about 79 mg to about 81 mg, about 79 mg to about 80 mg, about 80 mg to about 84 mg, about 80 mg to about 83 mg, about 80 mg to about 82 mg, about 80 mg to about 81 mg, about 81 mg to about 84 mg, about 81 mg to about 83 mg, about 81 mg to about 82 mg, about 82 mg to about 84 mg, about 82 mg to about 83 mg, or about 83 mg to about 84 mg tromethamine hydrochloride.

In various embodiments, the liquid pharmaceutical formulations described herein can comprise about 75 mg, about 76 mg, about 77 mg, about 78 mg, about 79 mg, about 80 mg, about 81 mg, about 82 mg, about 83 mg, about 84 mg, or about 85 mg tromethamine hydrochloride. In certain embodiments, the liquid pharmaceutical formulations described herein comprise about 79 mg tromethamine hydrochloride.

In various embodiments, the concentration of tromethamine hydrochloride in the liquid pharmaceutical formulation is about 25 mmol to about 500 mmol, about 50 mmol to about 500 mmol, about 100 mmol to about 500 mmol, about 150 mmol to about 500 mmol, about 200 mmol to about 500 mmol, about 250 mmol to about 500 mmol, about 300 mmol to about 500 mmol, about 400 mmol to about 500 mmol, about 25 mmol to about 400 mmol, about 25 mmol to about 300 mmol, about 25 mmol to about 250 mmol, about 25 mmol to about 200 mmol, about 25 mmol to about 150 mmol, about 25 mmol to about 100 mmol, about 25 mmol to about 50 mmol, about 50 mmol to about 400 mmol, about 50 mmol to about 300 mmol, about 50 mmol to about 250 mmol, about 50 mmol to about 200 mmol, about 50 mmol to about 150 mmol, about 50 mmol to about 100 mmol, about 100 mmol to about 400 mmol, about 100 mmol to about 300 mmol, about 100 mmol to about 250 mmol, about 100 mmol to about 200 mmol, about 100 mmol to about 150 mmol, about 150 mmol to about 400 mmol, about 150 mmol to about 300 mmol, about 150 mmol to about 250 mmol, about 150 mmol to about 200 mmol, about 200 mmol to about 400 mmol, about 200 mmol to about 300 mmol, about 200 mmol to about 250 mmol, about 250 mmol to about 400 mmol, about 250 mmol to about 300 mmol, or about 300 mmol to about 400 mmol.

In various embodiments, the concentration of tromethamine hydrochloride in the liquid pharmaceutical formulation is about 25 mmol, about 50 mmol, about 100 mmol, about 150 mmol, about 200 mmol, about 250 mmol, about 300 mmol, about 400 mmol, or about 500 mmol.

In various embodiments, the molar ratio of tromethamine hydrochloride to furosemide, or a pharmaceutically acceptable salt thereof, in the liquid pharmaceutical formulation is about 1 to about 3.5, about 1.5 to about 3.5, about 2 to about 3.5, about 2.5 to about 3.5, about 3 to about 3.5, about 1 to about 3, about 1 to about 2.5, about 1 to about 2, about 1 to about 1.5, about 1.5 to about 3, about 1.5 to about 2.5, about 1.5 to about 2, about 2 to about 3, about 2 to about 2.5, or about 2.5 to about 3.

In various embodiments, the molar ratio of tromethamine hydrochloride to furosemide, or a pharmaceutically acceptable salt thereof, in the liquid pharmaceutical formulation is about 1, about 1.5, about 2, about 2.5, about 3, or about 3.5.

In various embodiments, the molar ratio of tromethamine hydrochloride to furosemide in the liquid pharmaceutical formulation is about 1 to about 3.5, about 1.5 to about 3.5, about 2 to about 3.5, about 2.5 to about 3.5, about 3 to about 3.5, about 1 to about 3, about 1 to about 2.5, about 1 to about 2, about 1 to about 1.5, about 1.5 to about 3, about 1.5 to about 2.5, about 1.5 to about 2, about 2 to about 3, about 2 to about 2.5, or about 2.5 to about 3.

In various embodiments, the molar ratio of tromethamine hydrochloride to furosemide in the liquid pharmaceutical formulation is about 1, about 1.5, about 2, about 2.5, about 3, or about 3.5.

In various embodiments, the liquid pharmaceutical compositions described herein can comprise about 8 mL to about 12 mL, about 8.5 mL to about 12 mL, about 9 mL to about 12 mL, about 9.5 mL to about 12 mL, about 10 mL to about 12 mL, about 10.5 mL to about 12 mL, about 11 mL to about 12 mL, about 11.5 mL to about 12 mL, about 8 mL to about 11.5 mL, about 8 mL to about 11 mL, about 8 mL to about 10.5 mL, about 8 mL to about 10 mL, about 8 mL to about 9.5 mL, about 8 mL to about 9 mL, about 8 mL to about 8.5 mL, about 8.5 mL to about 11.5 mL, about 8.5 mL to about 11 mL, about 8.5 mL to about 10.5 mL, about 8.5 mL to about 10 mL, about 8.5 mL to about 9.5 mL, about 8.5 mL to about 9 mL, about 9 mL to about 11.5 mL, about 9 mL to about 11 mL, about 9 mL to about 10.5 mL, about 9 mL to about 10 mL, about 9 mL to about 9.5 mL, about 9.5 mL to about 11.5 mL, about 9.5 mL to about 11 mL, about 9.5 mL to about 10.5 mL, about 9.5 mL to about 10 mL, about 10 mL to about 11.5 mL, about 10 mL to about 11 mL, about 10 mL to about 10.5 mL, about 10.5 mL to about 11.5 mL, about 10.5 mL to about 11 mL, or about 11 mL to about 11.5 mL water.

In various embodiments, the liquid pharmaceutical compositions described herein can comprise about 8 mL, about 8.5 mL, about 9 mL, about 9.5 mL, about 10 mL, about 10.5 mL, about 11 mL, about 11.5 mL, or about 12 mL water. In certain embodiments, the liquid pharmaceutical compositions described herein can comprise about 10 mL water.

It should be understood that the volume of water added to the pharmaceutical formulation can be an amount to bring the total volume to one of the enumerated volumes of water herein, for example, to bring the total volume of the pharmaceutical formulation to 10 mL.

In various embodiments, the liquid pharmaceutical compositions described herein can comprise about 8 g to about 12 g, about 8.5 g to about 12 g, about 9 g to about 12 g, about 9.5 g to about 12 g, about 10 g to about 12 g, about 10.5 g to about 12 g, about 11 g to about 12 g, about 11.5 g to about 12 g, about 8 g to about 11.5 g, about 8 g to about 11 g, about 8 g to about 10.5 g, about 8 g to about 10 g, about 8 g to about 9.5 g, about 8 g to about 9 g, about 8 g to about 8.5 g, about 8.5 g to about 11.5 g, about 8.5 g to about 11 g, about 8.5 g to about 10.5 g, about 8.5 g to about 10 g, about 8.5 g to about 9.5 g, about 8.5 g to about 9 g, about 9 g to about 11.5 g, about 9 g to about 11 g, about 9 g to about 10.5 g, about 9 g to about 10 g, about 9 g to about 9.5 g, about 9.5 g to about 11.5 g, about 9.5 g to about 11 g, about 9.5 g to about 10.5 g, about 9.5 g to about 10 g, about 10 g to about 11.5 g, about 10 g to about 11 g, about 10 g to about 10.5 g, about 10.5 g to about 11.5 g, about 10.5 g to about 11 g, or about 11 g to about 11.5 g water.

In various embodiments, the liquid pharmaceutical compositions described herein can comprise about 8 g, about 8.5 g, about 9 g, about 9.5 g, about 10 g, about 10.5 g, about 11 g, about 11.5 g, or about 12 g water. In certain embodiments, the liquid pharmaceutical compositions described herein can comprise about 10 g water.

In various embodiments, the liquid pharmaceutical formulations described herein may further comprise one or more pharmaceutically acceptable excipients.

In various embodiments, a liquid pharmaceutical formulation comprises:
(i) furosemide, or a pharmaceutically acceptable salt thereof;
(ii) a pharmaceutically acceptable buffer: and
(iii) one or more pharmaceutically acceptable excipients.

In various embodiments, a liquid pharmaceutical formulation comprises:
(i) furosemide, or a pharmaceutically acceptable salt thereof;
(ii) a pharmaceutically acceptable buffer;
(iii) one or more pharmaceutically acceptable excipients; and
(iv) water.

In various embodiments, the one or more pharmaceutically acceptable excipients is selected from the group consisting of ethanol, benzyl alcohol, glycerin, N-methyl-pyrrolidone (NMP), sodium chloride, sodium hydroxide, hydrochloric acid, a polyethylene glycol (PEG), propylene glycol, a polysorbate, a polyvinylpyrrolidone (PVP), a cyclodextrin, and combinations thereof. In certain embodiments, the one or more pharmaceutically acceptable excipients is selected from the group consisting of sodium chloride, sodium hydroxide, hydrochloric acid, or combinations thereof. In certain embodiments, the liquid pharmaceutical formulations described herein further comprise sodium chloride, sodium hydroxide, hydrochloric acid, or combinations thereof.

In various embodiments, the liquid pharmaceutical formulations described herein have a pH of about 6.5 to about 8.5, about 7 to about 8.5, about 7.5 to about 8.5, about 8 to about 8.5, about 6.5 to about 8, about 6.5 to about 7.5, about 6.5 to about 7, about 7 to about 8, about 7 to about 7.5, or about 7.5 to about 8. In certain embodiments, the liquid pharmaceutical formulations described herein have a pH of about 7 to about 8.

In various embodiments, the liquid pharmaceutical formulations described herein have a pH of about 7 to about 8, about 7.2 to about 8, about 7.4 to about 8, about 7.6 to about 8, about 7.8 to about 8, about 7 to about 7.8, about 7 to about 7.6, about 7 to about 7.4, about 7 to about 7.2, about 7.2 to about 7.8, about 7.2 to about 7.6, about 7.2 to about 7.4, about 7.4 to about 7.8, about 7.4 to about 7.6, or about 7.6 to about 7.8. In certain embodiments, the liquid pharmaceutical formulations described herein have a pH of about 7 to about 7.8.

In various embodiments, the liquid pharmaceutical formulations described herein have a pH of about 6.5, about 7, about 7.5, about 8, or about 8.5.

In various embodiments, the liquid pharmaceutical formulations described herein have a pH of about 7, about 7.2, about 7.4, about 7.6, about 7.8, or about 8.

In various embodiments, a liquid pharmaceutical formulation comprises:
(i) furosemide, or a pharmaceutically acceptable salt thereof; and
(ii) a pharmaceutically acceptable buffer,
wherein the liquid pharmaceutical formulation has a pH of about 7 to about 7.8.

In various embodiments, a liquid pharmaceutical formulation comprises:
(i) furosemide, or a pharmaceutically acceptable salt thereof;
(ii) a pharmaceutically acceptable buffer: and
(iii) one or more pharmaceutically acceptable excipients,
wherein the liquid pharmaceutical formulation has a pH of about 7 to about 7.8.

In various embodiments, the total volume of the liquid pharmaceutical formulations described herein can be about 8 mL to about 12 mL, about 8.5 mL to about 12 mL, about 9 mL to about 12 mL, about 9.5 mL to about 12 mL, about 10 mL to about 12 mL, about 10.5 mL to about 12 mL, about 11 mL to about 12 mL, about 11.5 mL to about 12 mL, about 8 mL to about 11.5 mL, about 8 mL to about 11 mL, about 8 mL to about 10.5 mL, about 8 mL to about 10 mL, about 8 mL to about 9.5 mL, about 8 mL to about 9 mL, about 8 mL to about 8.5 mL, about 8.5 mL to about 11.5 mL, about 8.5 mL to about 11 mL, about 8.5 mL to about 10.5 mL, about 8.5 mL to about 10 mL, about 8.5 mL to about 9.5 mL, about 8.5 mL to about 9 mL, about 9 mL to about 11.5 mL, about 9 mL to about 11 mL, about 9 mL to about 10.5 mL, about 9 mL to about 10 mL. about 9 mL to about 9.5 mL, about 9.5 mL to about 11.5 mL, about 9.5 mL to about 11 mL, about 9.5 mL to about 10.5 mL, about 9.5 mL to about 10 mL, about 10 mL to about 11.5 mL, about 10 mL to about 11 mL, about 10 mL to about 10.5 mL, about 10.5 mL to about 11.5 mL, about 10.5 mL to about 11 mL, or about 11 mL to about 11.5 mL.

In various embodiments, the total volume of the liquid pharmaceutical formulations described herein can be about 8 mL, about 8.5 mL, about 9 mL, about 9.5 mL, about 10 mL, about 10.5 mL, about 11 mL, about 11.5 mL, or about 12 mL. In certain embodiments, the total volume of the liquid pharmaceutical formulation is about 10 mL.

In various embodiments, a liquid pharmaceutical formulation comprises about 80 mg furosemide. In certain embodiments, a liquid pharmaceutical formulation comprises about 80 mg furosemide and a total volume of about 10 mL.

In various embodiments, a liquid pharmaceutical formulation comprises:
(i) about 80 mg furosemide; and
(ii) about 79 mg tromethamine hydrochloride: and
(iii) water.

In various embodiments, a liquid pharmaceutical formulation comprises:
(i) about 80 mg furosemide: and
(ii) about 79 mg tromethamine hydrochloride; and
(iii) water,
wherein the liquid pharmaceutical formulation has a total volume of about 10 mL.

In various embodiments, a liquid pharmaceutical formulation comprises:
(i) about 80 mg furosemide;
(ii) about 79 mg tromethamine hydrochloride;
(iii) water; and
(iv) one or more pharmaceutically acceptable excipients.

In various embodiments, a liquid pharmaceutical formulation comprises:
(i) about 80 mg furosemide;
(ii) about 79 mg tromethamine hydrochloride;
(iii) water; and
(iv) one or more pharmaceutically acceptable excipients,
wherein the liquid pharmaceutical formulation has a total volume of about 10 mL.

In various embodiments, a liquid pharmaceutical formulation comprises:
(i) about 80 mg furosemide;
(ii) about 79 mg tromethamine hydrochloride:
(iii) water; and
(iv) one or more pharmaceutically acceptable excipients,
wherein the liquid pharmaceutical formulation has a pH of about 7 to about 7.8.

In various embodiments, a liquid pharmaceutical formulation comprises:
(i) about 80 mg furosemide;
(ii) about 79 mg tromethamine hydrochloride;
(iii) water; and
(iv) one or more pharmaceutically acceptable excipients,
wherein the liquid pharmaceutical formulation has a pH of about 7 to about 7.8 and a total volume of about 10 mL.

In various embodiments, a liquid pharmaceutical formulation comprises:
(i) about 80 mg furosemide:
(ii) about 79 mg tromethamine hydrochloride;
(iii) water: and
(iv) one or more pharmaceutically acceptable excipients selected from the group consisting of sodium chloride, sodium hydroxide, hydrochloric acid, and combinations thereof.

In various embodiments, a liquid pharmaceutical formulation comprises:
(i) about 80 mg furosemide:
(ii) about 79 mg tromethamine hydrochloride;
(iii) water; and
(iv) one or more pharmaceutically acceptable excipients selected from the group consisting of sodium chloride, sodium hydroxide, hydrochloric acid, and combinations thereof, wherein the liquid pharmaceutical formulation has a total volume of about 10 mL.

In various embodiments, a liquid pharmaceutical formulation comprises:
(i) about 80 mg furosemide;
(ii) about 79 mg tromethamine hydrochloride;
(iii) water; and
(iv) one or more pharmaceutically acceptable excipients selected from the group consisting of sodium chloride, sodium hydroxide, hydrochloric acid, and combinations thereof, wherein the liquid pharmaceutical formulation has a pH of about 7 to about 7.8.

In various embodiments, a liquid pharmaceutical formulation comprises:
(i) about 80 mg furosemide:
(ii) about 79 mg tromethamine hydrochloride;
(iii) water; and
(iv) one or more pharmaceutically acceptable excipients selected from the group consisting of sodium chloride, sodium hydroxide, hydrochloric acid, and combinations thereof,
wherein the liquid pharmaceutical formulation has a pH of about 7 to about 7.8 and a total volume of about 10 mL.

In various embodiments, the liquid pharmaceutical formulations described herein have an osmolarity in the range of about 250 mOsM (or 250 mOsm/kg) to about 350 mOsM (or 350 mOsm/kg), about 275 mOsM (or 275 mOsm/kg) to about 350 mOsM (or 350 mOsm/kg), about 300 mOsM (or 300 mOsm/kg) to about 350 mOsM (or 350 mOsm/kg), about 325 mOsM (or 325 mOsm/kg) to about 350 mOsM (or 350 mOsm/kg), about 250 mOsM (or 250 mOsm/kg) to about 325 mOsM (or 325 mOsm/kg), about 250 mOsM (or 250 mOsm/kg) to about 300 mOsM (or 300 mOsm/kg), about 250 mOsM (or 250 mOsm/kg) to about 275 mOsM (or 275 mOsm/kg), about 275 mOsM (or 275 mOsm/kg) to about 325 mOsM (or 325 mOsm/kg), about 275 mOsM (or 275 mOsm/kg) to about 300 mOsM (or 300 mOsm/kg), or about 300 mOsM (or 300 mOsm/kg) to about 325 mOsM (or 325 mOsm/kg).

In various embodiments, the liquid pharmaceutical formulations described herein are isosmotic.

In various embodiments, the liquid pharmaceutical formulations described herein further comprise a second therapeutic agent. In certain embodiments, furosemide is the sole therapeutic agent present in the liquid pharmaceutical formulations described herein.

In various embodiments, a liquid pharmaceutical formulation useful in the treatment of a condition, disease, or disorder described herein is a liquid pharmaceutical formulation described in U.S. Patent No. 9,884,039, which is incorporated by reference in its entirety herein.

### Methods of Treatment

In one aspect, the liquid pharmaceutical formulations described herein may be used for the treatment or prevention of a variety of conditions, diseases, and disorders such as, but not limited to, congestion, edema, fluid overload, or hypertension in a patient in need thereof. In various embodiments, the condition, disease, or disorder is selected from the group consisting of congestion, edema, fluid overload, hypertension, and combinations thereof. In certain embodiments, the condition, disease, or disorder is congestion due to fluid overload.

In various embodiments, the patient is an adult human.

In various embodiments, a method of treating a condition, disease, or disorder described herein in an adult human in need thereof, comprising administering subcutaneously a liquid pharmaceutical formulation described herein from a prefilled polymeric cartridge to the adult human over about 5 hours, wherein the furosemide concentration in plasma of the adult human is about 1200 ng/mL to about 1850 ng/mL after the first hour of administration, and about 1000 ng/mL to about 1800 ng/mL about one hour after administration is complete.

In various embodiments, a method of treating congestion due to fluid overload in an adult human in need thereof, comprising administering subcutaneously a liquid pharmaceutical formulation described herein from a prefilled polymeric cartridge to the adult human over about 5 hours, wherein the furosemide concentration in plasma of the adult human is about 1200 ng/mL to about 1850 ng/mL after the first hour of administration, and about 1000 ng/mL to about 1800 ng/mL about one hour after administration is complete.

In various embodiments, a total volume of about 8 mL to about 12 mL, about 8.5 mL to about 12 mL, about 9 mL to about 12 mL, about 9.5 mL to about 12 mL, about 10 mL to about 12 mL, about 10.5 mL to about 12 mL, about 11 mL to about 12 mL, about 11.5 mL to about 12 mL, about 8 mL to about 11.5 mL, about 8 mL to about 11 mL, about 8 mL to about 10.5 mL, about 8 mL to about 10 mL, about 8 mL to about 9.5 mL, about 8 mL to about 9 mL, about 8 mL to about 8.5 mL, about 8.5 mL to about 11.5 mL, about 8.5 mL to about 11 mL, about 8.5 mL to about 10.5 mL, about 8.5 mL to about 10 mL, about 8.5 mL to about 9.5 mL, about 8.5 mL to about 9 mL, about 9 mL to about 11.5 mL, about 9 mL to about 11 mL, about 9 mL to about 10.5 mL, about 9 mL to about 10 mL, about 9 mL to about 9.5 mL, about 9.5 mL to about 11.5 mL, about 9.5 mL to about 11 mL, about 9.5 mL to about 10.5 mL, about 9.5 mL to about 10 mL, about 10 mL to about 11.5 mL, about 10 mL to about 11 mL, about 10 mL to about 10.5 mL, about 10.5 mL to about 11.5 mL, about 10.5 mL to about 11 mL, or about 11 mL to about 11.5 mL of the liquid pharmaceutical formulation is administered over about 5 hours.

In various embodiments, a total volume of about 8 mL, about 8.5 mL, about 9 mL, about 9.5 mL, about 10 mL, about 10.5 mL, about 11 mL, about 11.5 mL, or about 12 mL of the liquid pharmaceutical formulation is administered over about 5 hours. In certain embodiments, a total volume of about 10 mL of the liquid pharmaceutical formulation is administered over about 5 hours.

In various embodiments, a method of treating a condition, disease, or disorder described herein in an adult human in need thereof, comprising administering subcutaneously a liquid pharmaceutical formulation described herein from a prefilled polymeric cartridge to the adult human over about 5 hours, wherein
a total volume of about 10 mL of the liquid pharmaceutical formulation is administered over about 5 hours; and
the furosemide concentration in plasma of the adult human is about 1200 ng/mL to about 1850 ng/mL after the first hour of administration, and about 1000 ng/mL to about 1800 ng/mL about one hour after administration is complete.

In various embodiments, a method of treating a condition, disease, or disorder described herein in an adult human in need thereof, comprising administering subcutaneously a liquid pharmaceutical formulation from a prefilled polymeric cartridge to the adult human over about 5 hours, wherein
the liquid pharmaceutical formulation comprises about 80 mg furosemide;
a total volume of about 10 mL of the liquid pharmaceutical formulation is administered over about 5 hours; and
the furosemide concentration in plasma of the adult human is about 1200 ng/mL to about 1850 ng/mL after the first hour of administration, and about 1000 ng/mL to about 1800 ng/mL about one hour after administration is complete.

In various embodiments, a method of treating congestion due to fluid overload in an adult human in need thereof, comprising administering subcutaneously a liquid pharmaceutical formulation described herein from a prefilled polymeric cartridge to the adult human over about 5 hours, wherein
a total volume of about 10 mL of the liquid pharmaceutical formulation is administered over about 5 hours; and
the furosemide concentration in plasma of the adult human is about 1200 ng/mL to about 1850 ng/mL after the first hour of administration, and about 1000 ng/mL to about 1800 ng/mL about one hour after administration is complete.

In various embodiments, a method of treating congestion due to fluid overload in an adult human in need thereof, comprising administering subcutaneously a liquid pharmaceutical formulation from a prefilled polymeric cartridge to the adult human over about 5 hours, wherein
the liquid pharmaceutical formulation comprises about 80 mg furosemide;
a total volume of about 10 mL of the liquid pharmaceutical formulation is administered over about 5 hours; and
the furosemide concentration in plasma of the adult human is about 1200 ng/mL to about 1850 ng/mL after the first hour of administration, and about 1000 ng/mL to about 1800 ng/mL about one hour after administration is complete.

In various embodiments, a method of treating a condition, disease, or disorder described herein in an adult human in need thereof, comprising administering subcutaneously a liquid pharmaceutical formulation described herein from a prefilled polymeric cartridge to the adult human over about 5 hours, wherein
a total volume of about 10 mL of the liquid pharmaceutical formulation is administered over about 5 hours; and
one or more of the following:
   the furosemide concentration in plasma of the adult human is about 1200 ng/mL to about 1850 ng/mL after the first hour of administration and about 1000 ng/mL to about 1800 ng/mL about one hour after administration is complete:
   the total urine output of the adult human during the first hour of administration is about 150 mL to about 450 mL and the average hourly urine output of the adult human one hour after administration is complete is about 250 mL to about 550 mL;
   the total urine output of the adult human about one hour after administration is complete is about 1500 mL to about 4000 mL;
   the total urine sodium excretion about one hour after administration is complete is about 375 mmol to about 500 mmol;
   the maximum total urine output of the adult human about one hour after administration is complete is about 2800 mL to about 4000 mL; and
   the maximum total urine sodium excretion about one hour after administration is complete is about 450 mmol to about 550 mmol.

In various embodiments, a method of treating a condition, disease, or disorder described herein in an adult human in need thereof, comprising administering subcutaneously a liquid pharmaceutical formulation from a prefilled polymeric cartridge to the adult human over about 5 hours, wherein
the liquid pharmaceutical formulation comprises about 80 mg furosemide;
a total volume of about 10 mL of the liquid pharmaceutical formulation is administered over about 5 hours; and
one or more of the following:
   the furosemide concentration in plasma of the adult human is about 1200 ng/mL to about 1850 ng/mL after the first hour of administration and about 1000 ng/mL to about 1800 ng/mL about one hour after administration is complete:
   the total urine output of the adult human during the first hour of administration is about 150 mL to about 450 mL and the average hourly urine output of the adult human one hour after administration is complete is about 250 mL to about 550 mL;
   the total urine output of the adult human about one hour after administration is complete is about 1500 mL to about 4000 mL;
   the total urine sodium excretion about one hour after administration is complete is about 375 mmol to about 500 mmol;
   the maximum total urine output of the adult human about one hour after administration is complete is about 2800 mL to about 4000 mL; and
   the maximum total urine sodium excretion about one hour after administration is complete is about 450 mmol to about 550 mmol.

In various embodiments, a method of treating a condition, disease, or disorder described herein in an adult human in need thereof, comprising administering subcutaneously a liquid pharmaceutical formulation described herein from a prefilled polymeric cartridge to the adult human over about 5 hours, wherein
a total volume of about 10 mL of the liquid pharmaceutical formulation is administered over about 5 hours;
the furosemide concentration in plasma of the adult human is about 1200 ng/mL to about 1850 ng/mL after the first hour of administration and about 1000 ng/mL to about 1800 ng/mL about one hour after administration is complete;
the total urine output of the adult human during the first hour of administration is about 150 mL to about 450 mL and the average hourly urine output of the adult human one hour after administration is complete is about 250 mL to about 550 mL;
the total urine output of the adult human about one hour after administration is complete is about 1500 mL to about 4000 mL;
the total urine sodium excretion about one hour after administration is complete is about 375 mmol to about 500 mmol;
the maximum total urine output of the adult human about one hour after administration is complete is about 2800 mL to about 4000 mL; and
the maximum total urine sodium excretion about one hour after administration is complete is about 450 mmol to about 550 mmol.

In various embodiments, a method of treating a condition, disease, or disorder described herein in an adult human in need thereof, comprising administering subcutaneously a liquid pharmaceutical formulation from a prefilled polymeric cartridge to the adult human over about 5 hours, wherein
the liquid pharmaceutical formulation comprises about 80 mg furosemide;
a total volume of about 10 mL of the liquid pharmaceutical formulation is administered over about 5 hours;
the furosemide concentration in plasma of the adult human is about 1200 ng/mL to about 1850 ng/mL after the first hour of administration and about 1000 ng/mL to about 1800 ng/mL about one hour after administration is complete;
the total urine output of the adult human during the first hour of administration is about 150 mL to about 450 mL and the average hourly urine output of the adult human one hour after administration is complete is about 250 mL to about 550 mL:
the total urine output of the adult human about one hour after administration is complete is about 1500 mL to about 4000 mL;
the total urine sodium excretion about one hour after administration is complete is about 375 mmol to about 500 mmol;
the maximum total urine output of the adult human about one hour after administration is complete is about 2800 mL to about 4000 mL; and
the maximum total urine sodium excretion about one hour after administration is complete is about 450 mmol to about 550 mmol.

In various embodiments, a method of treating congestion due to fluid overload in an adult human in need thereof, comprising administering subcutaneously a liquid pharmaceutical formulation described herein from a prefilled polymeric cartridge to the adult human over about 5 hours, wherein
a total volume of about 10 mL of the liquid pharmaceutical formulation is administered over about 5 hours: and
one or more of the following:
   the furosemide concentration in plasma of the adult human is about 1200 ng/mL to about 1850 ng/mL after the first hour of administration and about 1000 ng/mL to about 1800 ng/mL about one hour after administration is complete;
   the total urine output of the adult human during the first hour of administration is about 150 mL to about 450 mL and the average hourly urine output of the adult human one hour after administration is complete is about 250 mL to about 550 mL;
   the total urine output of the adult human about one hour after administration is complete is about 1500 mL to about 4000 mL;
   the total urine sodium excretion about one hour after administration is complete is about 375 mmol to about 500 mmol;
   the maximum total urine output of the adult human about one hour after administration is complete is about 2800 mL to about 4000 mL; and
   the maximum total urine sodium excretion about one hour after administration is complete is about 450 mmol to about 550 mmol.

In various embodiments, a method of treating congestion due to fluid overload in an adult human in need thereof, comprising administering subcutaneously a liquid pharmaceutical formulation from a prefilled polymeric cartridge to the adult human over about 5 hours, wherein
the liquid pharmaceutical formulation comprises about 80 mg furosemide;
a total volume of about 10 mL of the liquid pharmaceutical formulation is administered over about 5 hours; and
one or more of the following:
   the furosemide concentration in plasma of the adult human is about 1200 ng/mL to about 1850 ng/mL after the first hour of administration and about 1000 ng/mL to about 1800 ng/mL about one hour after administration is complete:
   the total urine output of the adult human during the first hour of administration is about 150 mL to about 450 mL and the average hourly urine output of the adult human one hour after administration is complete is about 250 mL to about 550 mL;
   the total urine output of the adult human about one hour after administration is complete is about 1500 mL to about 4000 mL;
   the total urine sodium excretion about one hour after administration is complete is about 375 mmol to about 500 mmol;
   the maximum total urine output of the adult human about one hour after administration is complete is about 2800 mL to about 4000 mL; and
   the maximum total urine sodium excretion about one hour after administration is complete is about 450 mmol to about 550 mmol.

In various embodiments, a method of treating congestion due to fluid overload in an adult human in need thereof, comprising administering subcutaneously a liquid pharmaceutical formulation described herein from a prefilled polymeric cartridge to the adult human over about 5 hours, wherein
a total volume of about 10 mL of the liquid pharmaceutical formulation is administered over about 5 hours;
the furosemide concentration in plasma of the adult human is about 1200 ng/mL to about 1850 ng/mL after the first hour of administration and about 1000 ng/mL to about 1800 ng/mL about one hour after administration is complete:
the total urine output of the adult human during the first hour of administration is about 150 mL to about 450 mL and the average hourly urine output of the adult human one hour after administration is complete is about 250 mL to about 550 mL;
the total urine output of the adult human about one hour after administration is complete is about 1500 mL to about 4000 mL;
the total urine sodium excretion about one hour after administration is complete is about 375 mmol to about 500 mmol;
the maximum total urine output of the adult human about one hour after administration is complete is about 2800 mL to about 4000 mL; and
the maximum total urine sodium excretion about one hour after administration is complete is about 450 mmol to about 550 mmol.

In various embodiments, a method of treating congestion due to fluid overload in an adult human in need thereof, comprising administering subcutaneously a liquid pharmaceutical formulation from a prefilled polymeric cartridge to the adult human over about 5 hours, wherein
the liquid pharmaceutical formulation comprises about 80 mg furosemide;
a total volume of about 10 mL of the liquid pharmaceutical formulation is administered over about 5 hours;
the furosemide concentration in plasma of the adult human is about 1200 ng/mL to about 1850 ng/mL after the first hour of administration and about 1000 ng/mL to about 1800 ng/mL about one hour after administration is complete;
the total urine output of the adult human during the first hour of administration is about 150 mL to about 450 mL and the average hourly urine output of the adult human one hour after administration is complete is about 250 mL to about 550 mL;
the total urine output of the adult human about one hour after administration is complete is about 1500 mL to about 4000 mL;
the total urine sodium excretion about one hour after administration is complete is about 375 mmol to about 500 mmol;
the maximum total urine output of the adult human about one hour after administration is complete is about 2800 mL to about 4000 mL; and
the maximum total urine sodium excretion about one hour after administration is complete is about 450 mmol to about 550 mmol.

In various embodiments, the liquid pharmaceutical formulation is administered from the prefilled polymeric cartridge to the adult human using a five-hour bi-phasic delivery profile.

In various embodiments, the five-hour bi-phasic delivery profile administers about 30 mg furosemide over the first hour and about 12.5 mg furosemide over each of the subsequent four hours.

In various embodiments, a method of treating a condition, disease, or disorder described herein in an adult human in need thereof, comprising administering subcutaneously a liquid pharmaceutical formulation from a prefilled polymeric cartridge to the adult human using a five-hour bi-phasic delivery profile, wherein
the liquid pharmaceutical formulation comprises about 80 mg furosemide, about 79 mg tromethamine hydrochloride and water, and has a pH between about 7 to about 7.8 and a total volume of 10 mL;
the five-hour bi-phasic delivery profile administers about 30 mg furosemide over the first hour and about 12.5 mg furosemide over each of the subsequent four hours, wherein the total volume of 10 mL of the liquid pharmaceutical formulation is administered over about five hours: and
one or more of the following:
   the furosemide concentration in plasma of the adult human is about 1200 ng/mL to about 1850 ng/mL after the first hour of administration and about 1000 ng/mL to about 1800 ng/mL about one hour after administration is complete;
   the total urine output of the adult human during the first hour of administration is about 150 mL to about 450 mL and the average hourly urine output of the adult human one hour after administration is complete is about 250 mL to about 550 mL;
   the total urine output of the adult human about one hour after administration is complete is about 1500 mL to about 4000 mL;
   the total urine sodium excretion about one hour after administration is complete is about 375 mmol to about 500 mmol;
   the maximum total urine output of the adult human about one hour after administration is complete is about 2800 mL to about 4000 mL; and
   the maximum total urine sodium excretion about one hour after administration is complete is about 450 mmol to about 550 mmol.

In various embodiments, a method of treating a condition, disease, or disorder described herein in an adult human in need thereof, comprising administering subcutaneously a liquid pharmaceutical formulation from a prefilled polymeric cartridge to the adult human using a five-hour bi-phasic delivery profile, wherein
the liquid pharmaceutical formulation comprises about 80 mg furosemide, about 79 mg tromethamine hydrochloride and water, and has a pH between about 7 to about 7.8 and a total volume of 10 mL;
the five-hour bi-phasic delivery profile administers about 30 mg furosemide over the first hour and about 12.5 mg furosemide over each of the subsequent four hours, wherein the total volume of 10 mL of the liquid pharmaceutical formulation is administered over about five hours;
the furosemide concentration in plasma of the adult human is about 1200 ng/mL to about 1850 ng/mL after the first hour of administration and about 1000 ng/mL to about 1800 ng/mL about one hour after administration is complete;
the total urine output of the adult human during the first hour of administration is about 150 mL to about 450 mL and the average hourly urine output of the adult human one hour after administration is complete is about 250 mL to about 550 mL;
the total urine output of the adult human about one hour after administration is complete is about 1500 mL to about 4000 mL;
the total urine sodium excretion about one hour after administration is complete is about 375 mmol to about 500 mmol;
the maximum total urine output of the adult human about one hour after administration is complete is about 2800 mL to about 4000 mL; and
the maximum total urine sodium excretion about one hour after administration is complete is about 450 mmol to about 550 mmol.

In various embodiments, a method of treating congestion due to fluid overload in an adult human in need thereof, comprising administering subcutaneously a liquid pharmaceutical formulation from a prefilled polymeric cartridge to the adult human using a five-hour bi-phasic delivery profile, wherein
the liquid pharmaceutical formulation comprises about 80 mg furosemide, about 79 mg tromethamine hydrochloride and water, and has a pH between about 7 to about 7.8 and a total volume of 10 mL:
the five-hour bi-phasic delivery profile administers about 30 mg furosemide over the first hour and about 12.5 mg furosemide over each of the subsequent four hours, wherein the total volume of 10 mL of the liquid pharmaceutical formulation is administered over about five hours; and
one or more of the following:
   the furosemide concentration in plasma of the adult human is about 1200 ng/mL to about 1850 ng/mL after the first hour of administration and about 1000 ng/mL to about 1800 ng/mL about one hour after administration is complete:
   the total urine output of the adult human during the first hour of administration is about 150 mL to about 450 mL and the average hourly urine output of the adult human one hour after administration is complete is about 250 mL to about 550 mL;
   the total urine output of the adult human about one hour after administration is complete is about 1500 mL to about 4000 mL;
   the total urine sodium excretion about one hour after administration is complete is about 375 mmol to about 500 mmol;
   the maximum total urine output of the adult human about one hour after administration is complete is about 2800 mL to about 4000 mL; and
   the maximum total urine sodium excretion about one hour after administration is complete is about 450 mmol to about 550 mmol.

In various embodiments, a method of treating congestion due to fluid overload in an adult human in need thereof, comprising administering subcutaneously a liquid pharmaceutical formulation from a prefilled polymeric cartridge to the adult human using a five-hour bi-phasic delivery profile, wherein
the liquid pharmaceutical formulation comprises about 80 mg furosemide, about 79 mg tromethamine hydrochloride and water, and has a pH between about 7 to about 7.8 and a total volume of 10 mL;
the five-hour bi-phasic delivery profile administers about 30 mg furosemide over the first hour and about 12.5 mg furosemide over each of the subsequent four hours, wherein the total volume of 10 mL of the liquid pharmaceutical formulation is administered over about five hours;
the furosemide concentration in plasma of the adult human is about 1200 ng/mL to about 1850 ng/mL after the first hour of administration and about 1000 ng/mL to about 1800 ng/mL about one hour after administration is complete;
the total urine output of the adult human during the first hour of administration is about 150 mL to about 450 mL and the average hourly urine output of the adult human one hour after administration is complete is about 250 mL to about 550 mL:
the total urine output of the adult human about one hour after administration is complete is about 1500 mL to about 4000 mL;
the total urine sodium excretion about one hour after administration is complete is about 375 mmol to about 500 mmol;
the maximum total urine output of the adult human about one hour after administration is complete is about 2800 mL to about 4000 mL; and
the maximum total urine sodium excretion about one hour after administration is complete is about 450 mmol to about 550 mmol.

In various embodiments, the furosemide concentration in plasma of the adult human is about 1200 ng/mL to about 1850 ng/mL, about 1250 ng/mL to about 1850 ng/mL, about 1300 ng/mL to about 1850 ng/mL, about 1350 ng/mL to about 1850 ng/mL, about 1400 ng/mL to about 1850 ng/mL, about 1450 ng/mL to about 1850 ng/mL, about 1500 ng/mL to about 1850 ng/mL, about 1550 ng/mL to about 1850 ng/mL, about 1600 ng/mL to about 1850 ng/mL, about 1650 ng/mL to about 1850 ng/mL, about 1700 ng/mL to about 1850 ng/mL, about 1750 ng/mL to about 1850 ng/mL, about 1800 ng/mL to about 1850 ng/mL, about 1200 ng/mL to about 1800 ng/mL, about 1200 ng/mL to about 1750 ng/mL, about 1200 ng/mL to about 1700 ng/mL, about 1200 ng/mL to about 1650 ng/mL, about 1200 ng/mL to about 1600 ng/mL, about 1200 ng/mL to about 1550 ng/mL, about 1200 ng/mL to about 1500 ng/mL, about 1200 ng/mL to about 1450 ng/mL, about 1200 ng/mL to about 1400 ng/mL, about 1200 ng/mL to about 1350 ng/mL, about 1200 ng/mL to about 1300 ng/mL, about 1200 ng/mL to about 1250 ng/mL, about 1250 ng/mL to about 1800 ng/mL, about 1250 ng/mL to about 1750 ng/mL, about 1250 ng/mL to about 1700 ng/mL, about 1250 ng/mL to about 1650 ng/mL, about 1250 ng/mL to about 1600 ng/mL, about 1250 ng/mL to about 1550 ng/mL, about 1250 ng/mL to about 1500 ng/mL, about 1250 ng/mL to about 1450 ng/mL, about 1250 ng/mL to about 1400 ng/mL, about 1250 ng/mL to about 1350 ng/mL, about 1250 ng/mL to about 1300 ng/mL, about 1300 ng/mL to about 1800 ng/mL, about 1300 ng/mL to about 1750 ng/mL, about 1300 ng/mL to about 1700 ng/mL, about 1300 ng/mL to about 1650 ng/mL, about 1300 ng/mL to about 1600 ng/mL, about 1300 ng/mL to about 1550 ng/mL, about 1300 ng/mL to about 1500 ng/mL, about 1300 ng/mL to about 1450 ng/mL, about 1300 ng/mL to about 1400 ng/mL, about 1300 ng/mL to about 1350 ng/mL, about 1350 ng/mL to about 1800 ng/mL, about 1350 ng/mL to about 1750 ng/mL, about 1350 ng/mL to about 1700 ng/mL, about 1350 ng/mL to about 1650 ng/mL, about 1350 ng/mL to about 1600 ng/mL, about 1350 ng/mL to about 1550 ng/mL, about 1350 ng/mL to about 1500 ng/mL, about 1350 ng/mL to about 1450 ng/mL, about 1350 ng/mL to about 1400 ng/mL, about 1400 ng/mL to about 1800 ng/mL, about 1400 ng/mL to about 1750 ng/mL, about 1400 ng/mL to about 1700 ng/mL, about 1400 ng/mL to about 1650 ng/mL, about 1400 ng/mL to about 1600 ng/mL, about 1400 ng/mL to about 1550 ng/mL, about 1400 ng/mL to about 1500 ng/mL, about 1400 ng/mL to about 1450 ng/mL, about 1450 ng/mL to about 1800 ng/mL, about 1450 ng/mL to about 1750 ng/mL, about 1450 ng/mL to about 1700 ng/mL, about 1450 ng/mL to about 1650 ng/mL, about 1450 ng/mL to about 1600 ng/mL, about 1450 ng/mL to about 1550 ng/mL, about 1450 ng/mL to about 1500 ng/mL, about 1500 ng/mL to about 1800 ng/mL, about 1500 ng/mL to about 1750 ng/mL, about 1500 ng/mL to about 1700 ng/mL, about 1500 ng/mL to about 1650 ng/mL, about 1500 ng/mL to about 1600 ng/mL, about 1500 ng/mL to about 1550 ng/mL, about 1550 ng/mL to about 1800 ng/mL, about 1550 ng/mL to about 1750 ng/mL, about 1550 ng/mL to about 1700 ng/mL, about 1550 ng/mL to about 1650 ng/mL, about 1550 ng/mL to about 1600 ng/mL, about 1600 ng/mL to about 1800 ng/mL, about 1600 ng/mL to about 1750 ng/mL, about 1600 ng/mL to about 1700 ng/mL, about 1600 ng/mL to about 1650 ng/mL, about 1650 ng/mL to about 1800 ng/mL, about 1650 ng/mL to about 1750 ng/mL, about 1650 ng/mL to about 1700 ng/mL, about 1700 ng/mL to about 1800 ng/mL, about 1700 ng/mL to about 1750 ng/mL, or about 1750 ng/mL to about 1800 ng/mL after the first hour of administration.

In certain embodiments, the furosemide concentration in plasma of the adult human is about 1200 ng/mL, about 1250 ng/mL, about 1300 ng/mL, about 1350 ng/mL, about 1400 ng/mL, about 1450 ng/mL, about 1500 ng/mL, about 1550 ng/mL, about 1600 ng/mL, about 1650 ng/mL, about 1700 ng/mL, about 1750 ng/mL, about 1800 ng/mL, or about 1850 ng/mL after the first hour of administration.

In various embodiments, the furosemide concentration in plasma of the adult human is about 1000 ng/mL to about 1800 ng/mL, about 1100 ng/mL to about 1800 ng/mL, about 1200 ng/mL to about 1800 ng/mL, about 1300 ng/mL to about 1800 ng/mL, about 1400 ng/mL to about 1800 ng/mL, about 1500 ng/mL to about 1800 ng/mL, about 1600 ng/mL to about 1800 ng/mL, about 1700 ng/mL to about 1800 ng/mL, about 1000 ng/mL to about 1700 ng/mL, about 1000 ng/mL to about 1600 ng/mL, about 1000 ng/mL to about 1500 ng/mL, about 1000 ng/mL to about 1400 ng/mL, about 1000 ng/mL to about 1300 ng/mL, about 1000 ng/mL to about 1200 ng/mL, about 1000 ng/mL to about 1100 ng/mL, about 1100 ng/mL to about 1700 ng/mL, about 1100 ng/mL to about 1600 ng/mL, about 1100 ng/mL to about 1500 ng/mL, about 1100 ng/mL to about 1400 ng/mL, about 1100 ng/mL to about 1300 ng/mL, about 1100 ng/mL to about 1200 ng/mL, about 1200 ng/mL to about 1700 ng/mL, about 1200 ng/mL to about 1600 ng/mL, about 1200 ng/mL to about 1500 ng/mL, about 1200 ng/mL to about 1400 ng/mL, about 1200 ng/mL to about 1300 ng/mL, about 1300 ng/mL to about 1700 ng/mL, about 1300 ng/mL to about 1600 ng/mL, about 1300 ng/mL to about 1500 ng/mL, about 1300 ng/mL to about 1400 ng/mL, about 1400 ng/mL to about 1700 ng/mL, about 1300 ng/mL to about 1600 ng/mL, about 1300 ng/mL to about 1500 ng/mL, about 1300 ng/mL to about 1400 ng/mL, about 1400 ng/mL to about 1700 ng/mL, about 1400 ng/mL to about 1600 ng/mL, about 1400 ng/mL to about 1500 ng/mL, about 1500 ng/mL to about 1700 ng/mL, about 1500 ng/mL to about 1600 ng/mL, or about 1600 ng/mL to about 1700 ng/mL, about one hour after administration is complete.

In various embodiments, the furosemide concentration in plasma of the adult human is about 1000 ng/mL, about 1100 ng/mL, about 1200 ng/mL, about 1300 ng/mL, about 1400 ng/mL, about 1500 ng/mL, about 1600 ng/mL, about 1700 ng/mL, or about 1800 ng/mL, about one hour after administration is complete.

In various embodiments, the furosemide concentration in plasma of the adult human is equal to or greater than 500 ng/mL, equal to or greater than 600 ng/mL, equal to or greater than 700 ng/mL, equal to or greater than 800 ng/mL, equal to or greater than 900 ng/mL, equal to or greater than 1000 ng/mL, equal to or greater than 1100 ng/mL, equal to or greater than 1200 ng/mL, equal to or greater than 1300 ng/mL, equal to or greater than 1400 ng/mL, equal to or greater than 1500 ng/mL, equal to or greater than 1600 ng/mL, equal to or greater than 1700 ng/mL, or equal to or greater than 1800 ng/mL after the first hour of administration to about one hour after administration is complete. In certain embodiments, the furosemide concentration in plasma of the adult human is equal to or greater than 1000 ng/mL after the first hour of administration to about one hour after administration is complete.

In various embodiments, the furosemide concentration in plasma of the adult human is equal to or greater than 500 ng/mL, equal to or greater than 600 ng/mL, equal to or greater than 700 ng/mL, equal to or greater than 800 ng/mL, equal to or greater than 900 ng/mL, equal to or greater than 1000 ng/mL, equal to or greater than 1100 ng/mL, equal to or greater than 1200 ng/mL, equal to or greater than 1300 ng/mL, equal to or greater than 1400 ng/mL, or equal to or greater than 1500 ng/mL, after the first hour of administration to about three hours after administration is complete.

In various embodiments, the total urine output of the adult human during the first hour of administration is about 150 mL to about 450 mL, about 200 mL to about 450 mL, about 250 mL to about 450 mL, about 300 mL to about 450 mL, about 350 mL to about 450 mL, about 400 mL to about 450 mL, about 150 mL to about 400 mL, about 150 mL to about 350 mL, about 150 mL to about 300 mL, about 150 mL to about 250 mL, about 150 mL to about 200 mL, about 200 mL to about 400 mL, about 200 mL to about 350 mL, about 200 mL to about 300 mL, about 200 mL to about 250 mL, about 250 mL to about 400 mL, about 250 mL to about 350 mL, about 250 mL to about 300 mL, about 300 mL to about 400 mL, about 350 mL to about 400 mL, or about 350 mL to about 400 mL.

In various embodiments, the total urine output of the adult human during the first hour of administration is about 150 mL, about 200 mL, about 250 mL, about 300 mL, about 350 mL, about 400 mL, or about 450 mL.

In various embodiments, the total urine output of the adult human during the first hour of administration is equal to or greater than 50 mL, equal to or greater than 75 mL, equal to or greater than 100 mL, equal to or greater than 125 mL, equal to or greater than 150 mL, equal to or greater than 175 mL, equal to or greater than 200 mL, equal to or greater than 225 mL, equal to or greater than 250 mL, equal to or greater than 275 mL, equal to or greater than 300 mL, equal to or greater than 325 mL, equal to or greater than 350 mL, equal to or greater than 375 mL, equal to or greater than 400 mL, or equal to or greater than 425 mL.

In various embodiments, the average hourly urine output of the adult human one hour after administration is complete is about 250 mL to about 500 mL, about 300 mL to about 500 mL, about 350 mL to about 500 mL, about 400 mL to about 500 mL, about 450 mL to about 500 mL, about 250 mL to about 450 mL, about 250 mL to about 400 mL, about 250 mL to about 350 mL, about 250 mL to about 300 mL, about 300 mL to about 450 mL, about 300 mL to about 400 mL, about 300 mL to about 350 mL, about 350 mL to about 450 mL, about 350 mL to about 400 mL, or about 400 mL to about 450 mL.

In various embodiments, the average hourly urine output of the adult human one hour after administration is complete is about 250 mL, about 300 mL, about 350 mL, about 400 mL, about 450 mL, or about 500 mL.

In various embodiments, the average hourly urine output of the adult human one hour after administration is complete is equal to or greater than 200 mL, equal to or greater than 225 mL, equal to or greater than 250 mL, equal to or greater than 275 mL, equal to or greater than 300 mL, equal to or greater than 325 mL, equal to or greater than 350 mL, equal to or greater than 375 mL, equal to or greater than 400 mL, equal to or greater than 425 mL, or equal to or greater than 450 mL.

In various embodiments, the total urine output of the adult human about one hour after administration is complete is about 1500 mL to about 4000 mL, about 2000 mL to about 4000 mL, about 2500 mL to about 4000 mL, about 3000 mL to about 4000 mL, about 3500 mL to about 4000 mL, about 1500 mL to about 3500 mL, about 1500 mL to about 3000 mL, about 1500 mL to about 2500 mL, about 1500 mL to about 2000 mL, about 2000 mL to about 3500 mL, about 2000 mL to about 3000 mL, about 2000 mL to about 2500 mL, about 2500 mL to about 3500 mL, about 2500 mL to about 3000 mL, or about 3000 mL to about 3500 mL.

In various embodiments, the total urine output of the adult human about one hour after administration is complete is about 1500 mL, about 2000 mL, about 2500 mL, about 3000 mL, about 3500 mL, or about 4000 mL.

In various embodiments, the total urine output of the adult human about one hour after administration is complete is equal to or greater than 1000 mL, equal to or greater than 1100 mL, equal to or greater than 1200 mL, equal to or greater than 1300 mL, equal to or greater than 1400 mL, equal to or greater than 1500 mL, equal to or greater than 1600 mL, equal to or greater than 1700 mL, equal to or greater than 1800 mL, equal to or greater than 1900 mL, equal to or greater than 2000 mL, equal to or greater than 2100 mL, equal to or greater than 2200 mL, equal to or greater than 2300 mL, equal to or greater than 2400 mL, equal to or greater than 2500 mL, equal to or greater than 2600 mL, equal to or greater than 2700 mL, equal to or greater than 2800 mL, equal to or greater than 2900 mL, equal to or greater than 3000 mL, equal to or greater than 3100 mL, equal to or greater than 3200 mL, equal to or greater than 3300 mL, equal to or greater than 3400 mL, equal to or greater than 3500 mL, equal to or greater than 3600 mL, equal to or greater than 3700 mL, equal to or greater than 3800 mL, or equal to or greater than 3900 mL.

In various embodiments, the total urine sodium excretion about one hour after administration is complete is about 375 mmol to about 500 mmol, about 400 mmol to about 500 mmol, about 425 mmol to about 500 mmol, about 450 mmol to about 500 mmol, about 475 mmol to about 500 mmol, about 375 mmol to about 475 mmol, about 375 mmol to about 450 mmol, about 375 mmol to about 425 mmol, about 425 mmol to about 475 mmol, about 425 mmol to about 450 mmol, or about 450 mmol to about 475 mmol.

In various embodiments, the total urine sodium excretion about one hour after administration is complete is about 375 mmol, about 400 mmol, about 425 mmol, about 450 mmol, about 475 mmol, or about 500 mmol.

In various embodiments, the total urine sodium excretion about one hour after administration is complete is equal to or greater than 300 mmol, equal to or greater than 325 mmol, equal to or greater than 350 mmol, equal to or greater than 375 mmol, equal to or greater than 400 mmol, equal to or greater than 425 mmol, equal to or greater than 450 mmol, or equal to or greater than 475 mmol.

In various embodiments, the maximum total urine output of the adult human about one hour after administration is complete is about 2800 mL to about 4000 mL, about 2900 mL to about 4000 mL, about 3000 mL to about 4000 mL, about 3100 mL to about 4000 mL, about 3200 mL to about 4000 mL, about 3300 mL to about 4000 mL, about 3400 mL to about 4000 mL, about 3500 mL to about 4000 mL, about 3600 mL to about 4000 mL, about 3700 mL to about 4000 mL, about 3800 mL to about 4000 mL, about 3900 mL to about 4000 mL, about 2800 mL to about 3900 mL, about 2800 mL to about 3800 mL, about 2800 mL to about 3700 mL, about 2800 mL to about 3600 mL, about 2800 mL to about 3500 mL, about 2800 mL to about 3400 mL, about 2800 mL to about 3300 mL, about 2800 mL to about 3200 mL, about 2800 mL to about 3100 mL, about 2800 mL to about 3000 mL, about 2800 mL to about 2900 mL, about 2900 mL to about 3900 mL, about 2900 mL to about 3800 mL, about 2900 mL to about 3700 mL, about 2900 mL to about 3600 mL, about 2900 mL to about 3500 mL, about 2900 mL to about 3400 mL, about 2900 mL to about 3300 mL, about 2900 mL to about 3200 mL, about 2900 mL to about 3100 mL, about 2900 mL to about 3000 mL, about 3000 mL to about 3900 mL, about 3000 mL to about 3800 mL, about 3000 mL to about 3700 mL, about 3000 mL to about 3600 mL, about 3000 mL to about 3500 mL, about 3000 mL to about 3400 mL, about 3000 mL to about 3300 mL, about 3000 mL to about 3200 mL, about 3000 mL to about 3100 mL, about 3100 mL to about 3900 mL, about 3100 mL to about 3800 mL, about 3100 mL to about 3700 mL, about 3100 mL to about 3600 mL, about 3100 mL to about 3500 mL, about 3100 mL to about 3400 mL, about 3100 mL to about 3300 mL, about 3100 mL to about 3200 mL, about 3200 mL to about 3900 mL, about 3200 mL to about 3800 mL, about 3200 mL to about 3700 mL, about 3200 mL to about 3600 mL, about 3200 mL to about 3500 mL, about 3200 mL to about 3400 mL, about 3200 mL to about 3300 mL, about 3300 mL to about 3900 mL, about 3300 mL to about 3800 mL, about 3300 mL to about 3700 mL, about 3300 mL to about 3600 mL, about 3300 mL to about 3500 mL, about 3300 mL to about 3400 mL, about 3400 mL to about 3900 mL, about 3400 mL to about 3800 mL, about 3400 mL to about 3700 mL, about 3400 mL to about 3600 mL, about 3400 mL to about 3500 mL, about 3500 mL to about 3900 mL, about 3500 mL to about 3800 mL, about 3500 mL to about 3700 mL, about 3500 mL to about 3600 mL, about 3600 mL to about 3900 mL, about 3600 mL to about 3800 mL, about 3600 mL to about 3700 mL, about 3700 mL to about 3900 mL, about 3700 mL to about 3800 mL, or about 3800 mL to about 3900 mL.

In various embodiments, the maximum total urine output of the adult human about one hour after administration is complete is about 2800 mL, about 2900 mL, about 3000 mL, about 3100 mL, about 3200 mL, about 3300 mL, about 3400 mL, about 3500 mL, about 3600 mL, about 3700 mL, about 3800 mL, about 3900 mL, or about 4000 mL.

In various embodiments, the maximum total urine output of the adult human about one hour after administration is complete is equal to or greater than 2000 mL, equal to or greater than 2100 mL, equal to or greater than 2200 mL, equal to or greater than 2300 mL, equal to or greater than 2400 mL, equal to or greater than 2500 mL, equal to or greater than 2600 mL, equal to or greater than 2700 mL, equal to or greater than 2800 mL, equal to or greater than 2900 mL, equal to or greater than 3000 mL, equal to or greater than 3100 mL, equal to or greater than 3200 mL, equal to or greater than 3300 mL, equal to or greater than 3400 mL, equal to or greater than 3500 mL, equal to or greater than 3600 mL, equal to or greater than 3700 mL, equal to or greater than 3800 mL, equal to or greater than 3900 mL, or equal to or greater than 4000 mL.

In various embodiments, the maximum total urine sodium excretion about one hour after administration is complete is about 450 mmol to about 550 mmol, about 475 mmol to about 550 mmol, about 500 mmol to about 550 mmol, about 525 mmol to about 550 mmol, about 450 mmol to about 525 mmol, about 450 mmol to about 500 mmol, about 450 mmol to about 475 mmol, about 475 mmol to about 525 mmol, about 475 mmol to about 500 mmol, or about 500 mmol to about 525 mmol.

In various embodiments, the maximum total urine sodium excretion about one hour after administration is complete is about 450 mmol, about 475 mmol, about 500 mmol, about 525 mmol, or about 550 mmol.

In various embodiments, the maximum total urine sodium excretion about one hour after administration is complete is equal to or greater than 400 mmol, equal to or greater than 425 mmol, equal to or greater than 450 mmol, equal to or greater than 475 mmol, equal to or greater than 500 mmol, or equal to or greater than 525 mmol.

In various embodiments, the method further comprises alerting the adult human when the total volume of 10 mL of the liquid pharmaceutical formulation is not administered during the five hours of the five-hour delivery profile. In certain embodiments, alerting comprises the adult human visualizing not all of the total volume of 10 mL of the liquid pharmaceutical formulation was delivered from the polymeric prefilled cartridge.

In various embodiments, the adult human has worsening New York Heart Association (NYHA) Class II and Class III heart failure.

In various embodiments, the adult human displays reduced responsiveness to oral diuretics.

### Kits

In one aspect, the invention provides kits for the treatment or prevention of a variety of conditions, diseases, and disorders such as, but not limited to, congestion, edema, fluid overload, or hypertension in a patient in need thereof. In certain embodiments, the condition, disease, or disorder is selected from the group consisting of congestion, edema, fluid overload, hypertension, and combinations thereof. In certain embodiments, the condition, disease, or disorder is congestion due to fluid overload.

In various embodiments, the invention provides a polymeric prefilled cartridge comprising a liquid pharmaceutical formulation described herein.

In various embodiments, the polymeric prefilled cartridge is associated with an on-body wearable delivery device.

In various embodiments, the polymeric prefilled cartridge is a single-use cartridge.

In various embodiments, a single-use polymeric prefilled cartridge that can be associated with an on-body wearable delivery device using a five-hour bi-phasic delivery profile for administering subcutaneously a liquid pharmaceutical formulation to an adult human, wherein the single-use polymeric prefilled cartridge comprises the liquid pharmaceutical formulation and the liquid pharmaceutical formulation is isosmotic, has a pH between about 7 to about 7.8, and consists of:
about 80 mg furosemide;
about 79 mg tromethamine hydrochloride:
   optionally, one or more other pharmaceutically acceptable excipients; and
   water to a total volume of 10 mL.

In various embodiments, the one or more other pharmaceutically acceptable excipients comprises sodium chloride, sodium hydroxide, hydrochloric acid, or combinations thereof.

In various embodiments, a kit includes instructions for use, for example, of a prefilled polymeric cartridge and an on-body wearable delivery device.

### EXAMPLES

The invention now being generally described, will be more readily understood by reference to the following examples, which are merely for the purposes of illustration of certain aspects and embodiments of the present invention, and are not intended to limit the invention.

### Example 1. Manufacturing Process for a Furosemide Formulation (8 mg/mL)

In the following example, the manufacturing process for preparing a 8 mg/mL furosemide liquid formulation is described.

The excipients (sodium chloride and tromethamine hydrochloride) and drug substance Furosemide USP/EP were dispensed into individual containers in amounts necessary to achieve final concentrations of furosemide, sodium chloride, and tromethamine hydrochloride of 8 mg/mL, 5.84 mg/mL, and 7.88 mg/mL, respectively.

The specified amount of water for injection (WFI) and the excipients, tromethamine hydrochloride and sodium chloride, were added to an appropriately sized mixing vessel. The vessel was closed, and the excipients were mixed. Sodium hydroxide (NaOH) solution was added to the vessel and mixed.

A pH sample was taken, and the solution adjusted with hydrochloric acid (HCl) or additional NaOH to a pH of 8.50 ± 0.10, if necessary. If an adjustment was necessary, the solution was mixed after each addition before sampling again.

The furosemide USP/NF/EP was then added to the excipient solution followed by a WFI rinse of the dispensing container. The vessel was closed, a lid was placed on the vessel to protect the drug substance from light, and the solution was mixed. After mixing, if the API had not dissolved, NaOH solution was added to the solution to facilitate dissolution.

A pH sample was taken, and the solution adjusted with HCl or additional NaOH solution to a pH of 7.40 ± 0.10.

After pH adjustments, WFI was added to the resulting solution to achieve the final weight. Samples were collected after final mixing for assay, pH, osmolality and appearance testing before continuing to filtration.

The furosemide liquid formulation was first filtered through a 0.45 µm bioburden reduction filter, and then a sterile filtration through dual 0.22 µm was performed. The furosemide liquid formulation was then filled into 10 mL polymeric cartridges and stoppered using an aseptic process.

### Example 2. Pharmacokinetic and Pharmacodynamic Characteristics of Subcutaneous Administration of Furosemide Liquid Formulations

### Study Objectives

The objectives of this study were to characterize the pharmacokinetics of furosemide administered by continuous subcutaneous infusion using a biphasic delivery profile: and to estimate the absolute bioavailability of furosemide administered by continuous subcutaneous infusion compared with an equivalent dose of furosemide administered by intravenous bolus administration.

### Study Design and Duration

This study was an open-label, single-center, single-dose, randomized, two-way (two-period) crossover study in 16 adult subjects previously diagnosed with mild to moderate heart failure (NYHA class II/III) being treated concomitantly with oral furosemide therapy at a dose of ≥ 40 mg/day.

Each subject completed screening, baseline, treatment, and follow-up phases. The screening phase was conducted on an outpatient basis between 14 and 3 days prior to baseline. Subjects were instructed to maintain a < 2 gm sodium diet within 3 days prior to Baseline. Baseline (Day 0) consisted of clinical research unit (CRU) admission and final qualification assessments. The treatment phase comprised two crossover periods separated by a 7-day outpatient fluid re-equilibration washout. Following CRU admission, subjects discontinued oral furosemide at least 24 hours prior to administration of study drug for each crossover period. Subjects were randomly assigned in a 1:1 ratio to 1 of 2 treatment sequences to receive both intravenous (IV) and subcutaneous (SC) furosemide in crossover periods (i.e., IV followed by SC or vice versa). Subjects remained domiciled in the CRU for each crossover period during the treatment phase through 24 hours after administration of study drug, after which time they were discharged if safety parameters were acceptable to the Investigator. Oral furosemide therapy was re-initiated at discharge after crossover period 1 (i.e., during the 7-day fluid re-equilibration washout) and after crossover period 2. The follow-up phase occurred 7 days (± 1) after discharge from the CRU following crossover period 2, completing subjects' study participation.

### Study Drug

Intravenous furosemide (IV Furosemide): Hospira, furosemide injection, solution 10 mg/mL (NDA 018667) (total dose = 80 mg) administered intravenously 40 mg over 2 minutes by IV bolus injection followed by a second dose of 40 mg over 2 minutes 120 minutes later (reference treatment).

Subcutaneous furosemide (SC furosemide): Furosemide injection solution, 8 mg/mL, (total dose = 80 mg dose) administered subcutaneously as 30 mg over the first hour and then as 12.5 mg per hour over the subsequent 4 hours (test treatment).

Study drug was administered at time zero (0) for the purpose of documenting drug administration, PK plasma sampling, urine collection, and AE monitoring. Serial sampling of venous blood for quantitation of furosemide in plasma was collected over 24 hours according to the schedule of procedures. Urine was collected from spontaneous voids in 1-hr increments for the first 2 hours, in 2-hour increments up to 12 hours post-dose and in 6-hour increments, thereafter, up to 24 hours post-dose for the purpose of determining the total urine volume and sodium concentration over time. Plasma samples were processed and stored according to the instructions in the protocol and assayed using validated bioanalytical methods.

### Number of Subjects

The number of subjects in this crossover study (i.e., n = 16) was intended to provide reasonably precise estimates of the systemic exposure of furosemide following IV and SC Furosemide treatments. In one study, the average coefficient of variation for furosemide AUCinf following a 20 mg dose by the intravenous, oral, and sublingual routes of administration were 50%, 25%, and 33%, respectively (Haegeli, L., et al., Sublingual administration of furosemide: new application of an old drug. Br J Clin Pharmacol., 2007. 64(6): p. 804-9. Epub 2007 Sep 13). Assuming that the relative bioavailability is equal to unity and that the intrasubject variability for AUC would fall within 25 to 50%, the estimated 90% CIs for the ratio of AUC between the test and reference treatments using sample sizes ranging from 12-16 subjects. A minimum of 12 subjects were expected to complete the study. If the number of subjects completing the study approaches the minimum number, additional subjects were to be enrolled at the discretion of the sponsor and investigator.

### Subject Population

Subjects were enrolled that had a history of at least 3 months treated heart failure (NYHA class II/III) who require ongoing treatment with oral furosemide at a dose of ≥ 40 mg per day for at least 30 days.

Male and female subjects aged ≥ 18 years.

Symptoms of chronic volume overload requiring oral furosemide ≥ 40 mg for at least 30 days.

No hospitalization for acute decompensated heart failure (ADHF) in past 4 weeks.

Subjects discontinued oral furosemide > 24 hours prior to administration of study drug.

### Pharmacokinetic Analyses

Individual pharmacokinetic parameters for furosemide were summarized with descriptive statistics. Pharmacokinetic parameters such as the maximum observed plasma concentration (Cmax), time to Cmax (Tmax), the area under the plasma concentration versus time curve from time 0 (predose) to the last quantifiable time point (AUClast), AUC from time 0 (predose) to time infinity (AUCinf), the elimination rate constant (λz) terminal elimination half-life (t½) for both SC Furosemide (test) and IV Furosemide (reference), and the apparent systematic clearance (CL/F) for SC furosemide and systematic clearance (CL) for IV Furosemide, the apparent systematic volume of distribution (Vz/F) for SC Furosemide and systematic volume of distribution (V) for IV Furosemide, were calculated using noncompartmental analysis. Absolute bioavailability of SC furosemide was calculated based on AUCinf of IV Furosemide (reference) and SC Furosemide (test). Compartmental modeling of the pharmacokinetic data was conducted as required.

### Results

Table 1 summarizes the individual efficacy response data (urine output (mL) hours 1, 6, and 8 after administration; cumulative urine output (mL) hours 6 and 8 after administration: cumulative sodium excretion (mmol) hours 6 and 8 after administration; plasma furosemide concentration (ng/mL) hours 1, 5, 6, and 8 after administration) for patients administered SC furosemide (80 mg). Table 2 summarizes the individual efficacy response data (urine output (mL) hours 1, 6, and 8 after administration; cumulative urine output (mL) hours 6 and 8 after administration; cumulative sodium excretion (mmol) hours 6 and 8 after administration; plasma furosemide concentration (ng/mL) hours 1, 5, 6, and 8 after administration) for patients administered IV furosemide (2-40 mg intravenous injections).

**Table 1. Individual Efficacy Response Data for Patients Administered Furosemide (80 mg) as 5-hour Subcutaneous Infusion**

| **Subject No.** | **Hour 1 Urine Output (mL)** | **Average Hourly Urine Output at Hour 6 (mL)** | **Average Hourly Urine Output at Hour 8 (mL)** | **Cumulative 6 hour urine output (mL)** | **Cumulative 8 hour urine output (mL)** |
|---|---|---|---|---|---|
| 1 | 60 | 141 | 137 | 845 | 1095 |
| 2 | 400 | 317 | 316 | 1900 | 2525 |
| 3 | 400 | 432 | 418 | 2590 | 3340 |
| 5 | 410 | 614 | 498 | 3685 | 3985 |
| 6 | 375 | 604 | 497 | 3625 | 3975 |
| 7 | 500 | 525 | 450 | 3150 | 3600 |
| 8 | 500 | 525 | 438 | 3150 | 3500 |
| 9 | 130 | 438 | 360 | 2630 | 2880 |
| 10 | 250 | 283 | 275 | 1700 | 2200 |
| 11 | 400 | 425 | 356 | 2550 | 2850 |
| 12 | 90 | 215 | 224 | 1290 | 1790 |
| 13 | 300 | 417 | 413 | 2500 | 3300 |
| 14 | 60 | 163 | 145 | 980 | 1160 |
| 15 | 175 | 254 | 203 | 1525 | 1625 |
| 16 | 200 | 200 | 178 | 1200 | 1425 |
| 17 | 250 | 450 | 403 | 2700 | 3220 |
| | | | | | |
| **Mean** | 281 | 375 | 332 | 2251 | 2654 |
| **Median** | 275 | 421 | 358 | 2525 | 2865 |
| **Min** | 60 | 141 | 137 | 845 | 1095 |
| **Max** | 500 | 614 | 498 | 3685 | 3985 |
| **90^{th} %** | 455 | 565 | 473 | 3388 | 3788 |
| **75^{th} %** | 400 | 469 | 423 | 2813 | 3380 |
| **50^{th} %** | 278 | 423 | 359 | 2538 | 2873 |
| **25^{th} %** | 164 | 244 | 219 | 1466 | 1749 |

**Table 1 (Continued). Individual Efficacy Response Data for Patients Administered Furosemide (80 mg) as 5-hour Subcutaneous Infusion**

| **Subject No.** | **Cumulative 6 hour sodium excretion (mmol)** | **Cumulative 8 hour sodium excretion (mmol)** | **1-hour Furosemide plasma conc. (ng/mL)** | **5-hour Furosemide plasma conc. (ng/mL)** | **6-hour Furosemide plasma conc. (ng/mL)** | **8-hour Furosemide plasma conc. (ng/mL)** |
|---|---|---|---|---|---|---|
| 1 | 159 | 203 | 1330 | 1930 | 1460 | 757 |
| 2 | 406 | 510 | 1820 | 2230 | 1550 | 644 |
| 3 | 491 | 589 | 985 | 1740 | 902 | 360 |
| 5 | 391 | 474 | 1430 | 1710 | 1070 | 604 |
| 6 | 503 | 619 | 1180 | 795 | 532 | 183 |
| 7 | 520 | 639 | 1110 | 1070 | 768 | 247 |
| 8 | 484 | 575 | 1370 | 1160 | 731 | 232 |
| 9 | 430 | 529 | 1220 | 1740 | 1090 | 450 |
| 10 | 376 | 446 | 1670 | 2780 | 2180 | 1070 |
| 11 | 481 | 578 | 1850 | 1940 | 1160 | 440 |
| 12 | 484 | 579 | 1800 | 2130 | 1080 | 454 |
| 13 | 344 | 439 | 1580 | 2230 | 1630 | 942 |
| 14 | 481 | 598 | 976 | 2200 | 1670 | 879 |
| 15 | 487 | 590 | 1470 | 2690 | 1530 | 758 |
| 16 | 382 | 424 | 1780 | 2580 | 1920 | 853 |
| 17 | 364 | 436 | 1950 | 1580 | 988 | 235 |
| | | | | | | |
| **Mean** | 424 | 514 | 1470 | 1907 | 1266 | 569 |
| **Median** | 456 | 552 | 1450 | 1935 | 1125 | 529 |
| **Min** | 159 | 203 | 976 | 795 | 532 | 183 |
| **Max** | 520 | 639 | 1950 | 2780 | 2180 | 1070 |
| **90^{th} %** | 497 | 609 | 1835 | 2635 | 1795 | 911 |
| **75^{th} %** | 485 | 589 | 1785 | 2230 | 1570 | 782 |
| **50^{th} %** | 468 | 564 | 1460 | 1921 | 1108 | 492 |
| **25^{th} %** | 381 | 444 | 1210 | 1475 | 933 | 244 |

**Table 2. Individual Efficacy Response Data for Patients Administered Furosemide (80 mg) as 2-40 mg Intravenous Injections**

| **Subject No.** | **Hour 1 Urine Output (mL)** | **Average Hourly Urine Output at Hour 6 (mL)** | **Average Hourly Urine Output at Hour 8 (mL)** | **Cumulative 6 hour urine output (mL)** | **Cumulative 8 hour urine output (mL)** |
|---|---|---|---|---|---|
| 1 | 300 | 230 | 223 | 1380 | 1780 |
| 2 | 80 | 148 | 124 | 890 | 990 |
| 3 | 425 | 329 | 303 | 1975 | 2425 |
| 5 | 750 | 575 | 481 | 3450 | 3850 |
| 6 | 900 | 550 | 433 | 3300 | 3460 |
| 7 | 1000 | 433 | 350 | 2600 | 2800 |
| 8 | 800 | 450 | 371 | 2700 | 2970 |
| 9 | 1050 | 567 | 438 | 3400 | 3500 |
| 10 | 750 | 355 | 291 | 2130 | 2330 |
| 11 | 850 | 425 | 338 | 2550 | 2700 |
| 12 | 1350 | 542 | 438 | 3250 | 3500 |
| 13 | 700 | 433 | 381 | 2600 | 3050 |
| 14 | 400 | 292 | 228 | 1750 | 1825 |
| 15 | 550 | 283 | 263 | 1700 | 2100 |
| 16 | 200 | 350 | 275 | 2100 | 2200 |
| 17 | 200 | 333 | 285 | 2000 | 2280 |
| | | | | | |
| **Mean** | 644 | 393 | 352 | 2579 | 2813 |
| **Median** | 725 | 390 | 350 | 2600 | 2800 |
| **Min** | 80 | 148 | 228 | 1700 | 1825 |
| **Max** | 1350 | 575 | 481 | 3450 | 3850 |
| **90^{th} %** | 1025 | 558 | 438 | 3380 | 3500 |
| **75^{th} %** | 863 | 473 | 381 | 2700 | 3050 |
| **50^{th} %** | 738 | 409 | 350 | 2579 | 2800 |
| **25^{th} %** | 513 | 346 | 275 | 2000 | 2200 |

**Table 2 (Continued). Individual Efficacy Response Data for Patients Administered Furosemide (80 mg) as 2-40 mg Intravenous Injections**

| **Subject No.** | **Cumulative 6 hour sodium excretion (mmol)** | **Cumulative 8 hour sodium excretion (mmol)** | **1-hour Furosemide plasma conc. (ng/mL)** | **5-hour Furosemide plasma conc. (ng/mL)** | **6-hour Furosemide plasma conc. (ng/mL)** | **8-hour Furosemide plasma conc. (ng/mL)** |
|---|---|---|---|---|---|---|
| 1 | 425 | 450 | 1910 | 1010 | 650 | 399 |
| 2 | 322 | 359 | 2110 | 929 | 602 | 279 |
| 3 | 407 | 447 | 1560 | 401 | 264 | 133 |
| 5 | 413 | 463 | 1620 | 724 | 481 | 247 |
| 6 | 435 | 547 | 920 | 270 | 132 | 80 |
| 7 | 530 | 652 | 1220 | 294 | 173 | 75 |
| 8 | 537 | 593 | 1190 | 240 | 131 | 60 |
| 9 | 524 | 659 | 1250 | 577 | 385 | 219 |
| 10 | 458 | 551 | 2580 | 1340 | 928 | 566 |
| 11 | 507 | 593 | 1860 | 654 | 397 | 178 |
| 12 | 518 | 635 | 1650 | 533 | 317 | 152 |
| 13 | 423 | 530 | 2210 | 1150 | 835 | 566 |
| 14 | 484 | 587 | 1570 | 919 | 636 | 328 |
| 15 | 425 | 504 | 2190 | 1330 | 907 | 532 |
| 16 | 408 | 486 | 1250 | 679 | 329 | 170 |
| 17 | 426 | 462 | 2220 | 467 | 322 | 200 |
| | | | | | | |
| **Mean** | 468 | 559 | 1672 | 706 | 459 | 260 |
| **Median** | 458 | 551 | 1620 | 654 | 385 | 200 |
| **Min** | 408 | 462 | 920 | 240 | 131 | 60 |
| **Max** | 537 | 659 | 2580 | 1340 | 928 | 566 |
| **90^{th} %** | 529 | 649 | 2218 | 1294 | 893 | 559 |
| **75^{th} %** | 518 | 593 | 2190 | 919 | 636 | 328 |
| **50^{th} %** | 468 | 559 | 1650 | 654 | 385 | 200 |
| **25^{th} %** | 425 | 504 | 1250 | 533 | 322 | 170 |

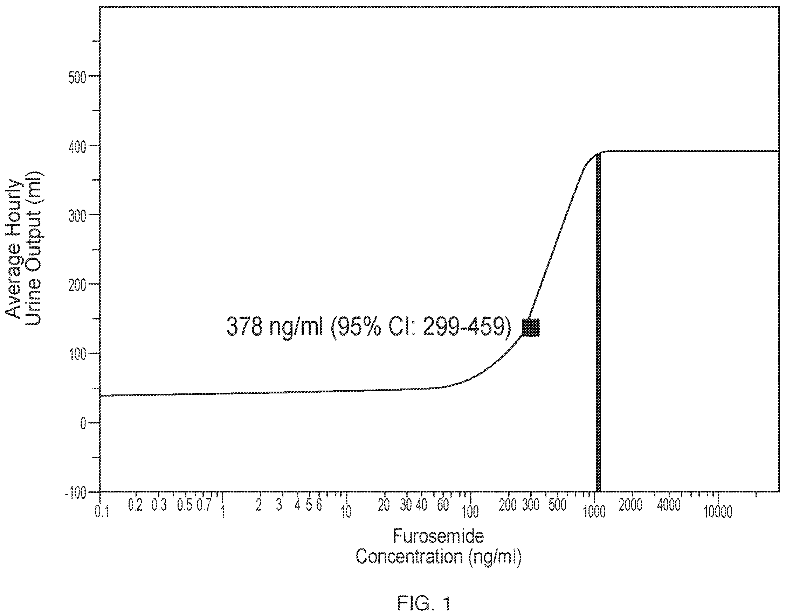

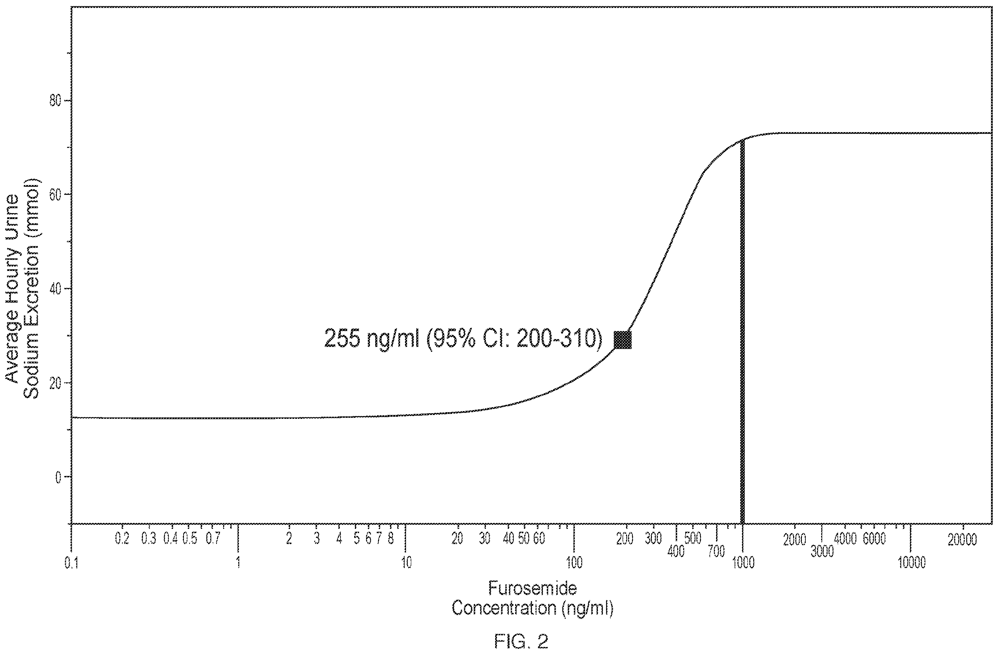

Further analysis of the individual efficacy response data for SC furosemide demonstrated that, surprisingly, both maximum hourly urine output and hourly sodium excretion were achieved in patients with a furosemide blood plasma concentration of about 1000 ng/mL (FIG. 1 and FIG. 2). Further increases in furosemide blood plasma concentration did not result in a corresponding increase in either hourly urine output or hourly sodium excretion.

### INCORPORATION BY REFERENCE

The entire disclosure of each of the patent documents and scientific articles referred to herein is incorporated by reference for all purposes.

### EQUIVALENTS

The disclosure may be embodied in other specific forms without departing from the spirit or essential characteristics thereof. The foregoing embodiments are therefore to be considered in all respects illustrative rather than limiting the disclosure described herein. Scope of the disclosure is thus indicated by the appended claims rather than by the foregoing description, and all changes that come within the meaning and range of equivalency of the claims are intended to be embraced therein.

Embodiments of the invention may include the features of the following enumerated paragraphs ("paras").
1. A method of treating congestion due to fluid overload in an adult human in need thereof, comprising administering subcutaneously a liquid pharmaceutical formulation from a prefilled polymeric cartridge to the adult human over about 5 hours, wherein
   the liquid pharmaceutical formulation comprises about 80 mg furosemide;
   a total volume of about 10 mL of the liquid pharmaceutical formulation is administered over about 5 hours; and
   the furosemide concentration in plasma of the adult human is about 1200 ng/mL to about 1850 ng/mL after the first hour of administration, and about 1000 ng/mL to about 1800 ng/mL about one hour after administration is complete.
2. The method of para 1, wherein the furosemide concentration in plasma of the adult human is equal to or greater than 1000 ng/mL after the first hour of administration to about one hour after administration is complete.
3. The method of para 1 or 2, wherein the total urine output of the adult human during the first hour of administration is about 150 mL to about 450 mL and the average hourly urine output of the adult human one hour after administration is complete is about 250 mL to about 550 mL.
4. The method of any one of para 1-3, wherein the total urine output of the adult human about one hour after administration is complete is about 1500 mL to about 4000 mL.
5. The method of any one of para 1-4, and the total urine sodium excretion about one hour after administration is complete is about 375 mmol to about 500 mmol.
6. The method of any one of paras 1-5, wherein the maximum total urine output of the adult human about one hour after administration is complete is about 2800 mL to about 4000 mL.
7. The method of any one of paras 1-6, wherein the maximum total urine sodium excretion about one hour after administration is complete is about 450 mmol to about 550 mmol.
8. A method of treating congestion due to fluid overload in an adult human in need thereof, comprising administering subcutaneously a liquid pharmaceutical formulation from a prefilled polymeric cartridge to the adult human over about 5 hours, wherein
   the liquid pharmaceutical formulation comprises about 80 mg furosemide;
   a total volume of about 10 mL of the liquid pharmaceutical formulation is administered over about 5 hours; and
   one or more of the following:
      the furosemide concentration in plasma of the adult human is about 1200 ng/mL to about 1850 ng/mL after the first hour of administration and about 1000 ng/mL to about 1800 ng/mL about one hour after administration is complete:
      the total urine output of the adult human during the first hour of administration is about 150 mL to about 450 mL and the average hourly urine output of the adult human one hour after administration is complete is about 250 mL to about 550 mL;
      the total urine output of the adult human about one hour after administration is complete is about 1500 mL to about 4000 mL;
      the total urine sodium excretion about one hour after administration is complete is about 375 mmol to about 500 mmol:
         the maximum total urine output of the adult human about one hour after administration is complete is about 2800 mL to about 4000 mL; and
         the maximum total urine sodium excretion about one hour after administration is complete is about 450 mmol to about 550 mmol.
9. A method of treating congestion due to fluid overload in an adult human in need thereof, comprising administering subcutaneously a liquid pharmaceutical formulation from a prefilled polymeric cartridge to the adult human over about 5 hours, wherein
   the liquid pharmaceutical formulation comprises about 80 mg furosemide;
   a total volume of about 10 mL of the liquid pharmaceutical formulation is administered over about 5 hours;
   the furosemide concentration in plasma of the adult human is about 1200 ng/mL to about 1850 ng/mL after the first hour of administration and about 1000 ng/mL to about 1800 ng/mL about one hour after administration is complete;
   the total urine output of the adult human during the first hour of administration is about 150 mL to about 450 mL and the average hourly urine output of the adult human one hour after administration is complete is about 250 mL to about 550 mL:
      the total urine output of the adult human about one hour after administration is complete is about 1500 mL to about 4000 mL;
      the total urine sodium excretion about one hour after administration is complete is about 375 mmol to about 500 mmol;
      the maximum total urine output of the adult human about one hour after administration is complete is about 2800 mL to about 4000 mL; and
      the maximum total urine sodium excretion about one hour after administration is complete is about 450 mmol to about 550 mmol.
10. The method of any one of paras 1-9, wherein the liquid pharmaceutical formulation is administered from the prefilled polymeric cartridge to the adult human using a five-hour biphasic delivery profile.
11. The method of para 10, wherein the five-hour bi-phasic delivery profile administers about 30 mg furosemide over the first hour and about 12.5 mg furosemide over each of the subsequent four hours.
12. The method of any one of paras 1-11, wherein the liquid pharmaceutical formulation further comprises a pharmaceutically acceptable buffer.
13. The method of para 12, wherein the pharmaceutically acceptable buffer comprises a buffering agent selected from the group consisting of histidine, a citrate salt, sodium phosphate, potassium phosphate, tromethamine or a pharmaceutically acceptable salt thereof, and combinations thereof.
14. The method of para 13, wherein the buffering agent is tromethamine hydrochloride.
15. The method of any one of paras 1-14, wherein the liquid pharmaceutical formulation further comprises about 79 mg tromethamine hydrochloride.
16. The method of any one of paras 1-15, wherein the liquid pharmaceutical formulation further comprises water.
17. The method of any one of paras 1-16, wherein the liquid pharmaceutical formulation has a pH of about 7 to about 7.8.
18. A method of treating congestion due to fluid overload in an adult human in need thereof, comprising administering subcutaneously a liquid pharmaceutical formulation from a prefilled polymeric cartridge to the adult human using a five-hour bi-phasic delivery profile, wherein
   the liquid pharmaceutical formulation comprises about 80 mg furosemide, about 79 mg tromethamine hydrochloride, and water, and has a pH between about 7 to about 7.8 and a total volume of 10 mL;
   the five-hour bi-phasic delivery profile administers about 30 mg furosemide over the first hour and about 12.5 mg furosemide over each of the subsequent four hours, wherein the total volume of 10 mL of the liquid pharmaceutical formulation is administered over about five hours; and
   one or more of the following:
      the furosemide concentration in plasma of the adult human is about 1200 ng/mL to about 1850 ng/mL after the first hour of administration and about 1000 ng/mL to about 1800 ng/mL about one hour after administration is complete;
      the total urine output of the adult human during the first hour of administration is about 150 mL to about 450 mL and the average hourly urine output of the adult human one hour after administration is complete is about 250 mL to about 550 mL;
      the total urine output of the adult human about one hour after administration is complete is about 1500 mL to about 4000 mL;
      the total urine sodium excretion about one hour after administration is complete is about 375 mmol to about 500 mmol;
      the maximum total urine output of the adult human about one hour after administration is complete is about 2800 mL to about 4000 mL; and
      the maximum total urine sodium excretion about one hour after administration is complete is about 450 mmol to about 550 mmol.
19. A method of treating congestion due to fluid overload in an adult human in need thereof, comprising administering subcutaneously a liquid pharmaceutical formulation from a prefilled polymeric cartridge to the adult human using a five-hour bi-phasic delivery profile, wherein
   the liquid pharmaceutical formulation comprises about 80 mg furosemide, about 79 mg tromethamine hydrochloride, and water, and has a pH between about 7 to about 7.8 and a total volume of 10 mL:
   the five-hour bi-phasic delivery profile administers about 30 mg furosemide over the first hour and about 12.5 mg furosemide over each of the subsequent four hours, wherein the total volume of 10 mL of the liquid pharmaceutical formulation is administered over about five hours;
   the furosemide concentration in plasma of the adult human is about 1200 ng/mL to about 1850 ng/mL after the first hour of administration and about 1000 ng/mL to about 1800 ng/mL about one hour after administration is complete:
   the total urine output of the adult human during the first hour of administration is about 150 mL to about 450 mL and the average hourly urine output of the adult human one hour after administration is complete is about 250 mL to about 550 mL;
   the total urine output of the adult human about one hour after administration is complete is about 1500 mL to about 4000 mL;
   the total urine sodium excretion about one hour after administration is complete is about 375 mmol to about 500 mmol;
   the maximum total urine output of the adult human about one hour after administration is complete is about 2800 mL to about 4000 mL: and
   the maximum total urine sodium excretion about one hour after administration is complete is about 450 mmol to about 550 mmol.
20. The method of any one of paras 1-19, wherein the liquid pharmaceutical composition further comprises one or more pharmaceutically acceptable excipients.
21. The method of para 20, wherein the one or more pharmaceutically acceptable excipients is selected from the group consisting of ethanol, benzyl alcohol, glycerin, N-methyl-pyrrolidone (NMP), sodium chloride, sodium hydroxide, hydrochloric acid, a polyethylene glycol (PEG), propylene glycol, a polysorbate, a polyvinylpyrrolidone (PVP), a cyclodextrin, and combinations thereof.
22. The method of any one of paras 1-19, wherein the liquid pharmaceutical formulation further comprises sodium chloride, sodium hydroxide, hydrochloric acid, or combinations thereof.
23. The method of any one of paras 1-22, wherein the liquid pharmaceutical formulation is isosmotic.
24. The method of any one of paras 1-23, wherein the polymeric prefilled cartridge is associated with an on-body wearable delivery device.
25. The method of any one of paras 1-24, wherein the polymeric cartridge of the polymeric prefilled cartridge comprises a cyclic olefin polymer.
26. The method of any one of paras 1-25, wherein the polymeric prefilled cartridge is a single-use cartridge.
27. The method of any one of paras 1-26, further comprising alerting the adult human when the total volume of 10 mL of the liquid pharmaceutical formulation is not administered during the five hours of the five-hour delivery profile.
28. The method of para 27, wherein alerting comprises the adult human visualizing not all of the total volume of 10 mL of the liquid pharmaceutical formulation was delivered from the polymeric prefilled cartridge.
29. The method of any one of paras 1-28, wherein the adult human has worsening New York Heart Association (NYHA) Class II and Class III heart failure.
30. The method of any one of paras 1-29, wherein the adult human displays reduced responsiveness to oral diuretics.
31. A single-use polymeric prefilled cartridge that can be associated with an on-body wearable delivery device using a five-hour bi-phasic delivery profile for administering subcutaneously a liquid pharmaceutical formulation to an adult human, wherein the single-use polymeric prefilled cartridge comprises the liquid pharmaceutical formulation and the liquid pharmaceutical formulation is isosmotic, has a pH between about 7 to about 7.8, and consists of:
   about 80 mg furosemide;
   about 79 mg tromethamine hydrochloride;
   optionally, one or more other pharmaceutically acceptable excipients; and
   water to a total volume of 10 mL.
32. The polymeric prefilled cartridge of para 31, wherein the one or more other pharmaceutically acceptable excipients comprises sodium chloride, sodium hydroxide, hydrochloric acid, or combinations thereof.

## Claims

1. A single-use polymeric prefilled cartridge that can be associated with an on-body wearable delivery device using a five-hour bi-phasic delivery profile for administering subcutaneously a liquid pharmaceutical formulation to an adult human, wherein the single-use polymeric prefilled cartridge comprises the liquid pharmaceutical formulation and the liquid pharmaceutical formulation is isosmotic, has a pH between about 7 to about 7.8, and comprises:
about 80 mg furosemide;
tromethamine hydrochloride;
one or more pharmaceutically acceptable excipients selected from the group consisting of ethanol, benzyl alcohol, glycerin, N-methyl-pyrrolidone (NMP), a polyethylene glycol (PEG), propylene glycol, a polysorbate, a polyvinylpyrrolidone (PVP), a cyclodextrin, and combinations thereof; and
water.

2. The single-use polymeric prefilled cartridge of claim 1, wherein the one or more pharmaceutically acceptable excipients comprises benzyl alcohol.

3. The single-use polymeric prefilled cartridge of claim 1 or 2, wherein the liquid pharmaceutical formulation further comprises sodium chloride, sodium hydroxide, hydrochloric acid, or combinations thereof.

4. The single-use polymeric prefilled cartridge of any one of claims 1-3, wherein the five-hour bi-phasic delivery profile administers about 30 mg furosemide over the first hour and about 12.5 mg furosemide over each of the subsequent four hours.

5. The single-use polymeric prefilled cartridge of any one of claims 1-4, wherein the single-use polymeric prefilled cartridge is associated with an on-body wearable delivery device.

6. The single-use polymeric prefilled cartridge of claim 5, wherein the on-body wearable delivery device is an auto-injection device.

7. The single-use polymeric prefilled cartridge of any one of claims 1-6 for use in the treatment or prevention of congestion, edema, fluid overload, hypertension, and combinations thereof.

8. The single-use polymeric prefilled cartridge of claim 7 for use in the treatment or prevention of congestion.

9. The single-use polymeric prefilled cartridge of claim 7 or 8 for use in the treatment or prevention of congestion due to fluid overload.

10. A kit comprising the single-use polymeric prefilled cartridge of any one of claims 1-6; and instructions for use.

11. The kit of claim 10, wherein the kit is for the treatment or prevention of congestion, edema, fluid overload, hypertension, and combinations thereof.

12. The kit of claim 10 or 11, wherein the kit is for the treatment or prevention of congestion.

13. The kit of any one of claims 10 to 12, wherein the kit is for the treatment or prevention of congestion due to fluid overload.
